# FASCICULE DE BREVET EUROPEEN

(11) **EP 2 854 801 B1**
(45) Date de publication et mention de la délivrance du brevet: **30.05.2018**
(21) Numéro de dépôt: 13725714.3
(22) Date de dépôt: 30.05.2013
(51) Int. Cl.: A61K 31/402, A61P 17/02, A61P 17/10, A61P 17/12, A61P 17/16, A61K 8/25, A61K 8/67, A61K 8/81

(54) **COMPOSITIONS TOPIQUES DE TYPE GEL AQUEUX SOUS FORME DE SUSPENSION HOMOGÈNE D'UN PRINCIPE ACTIF DE LA CLASSE DES RÉTINOÏDES CONTENANT AU MOINS UNE SILICE HYDROPHOBE**
TOPISCHE GELZUSAMMENSETZUNG ALS EINE HOMOGENE WÄSSRIGE SUSPENSION EINES RETINOIDS, DIE MINDESTENS EINE HYDROPHOBE KIESELSAÜRE ENTHÄLT
TOPICAL AQUEOUS GEL COMPOSITION IN THE FORM OF AN HOMOGENOUS SUSPENSION OF A RETINOID CONTAINING AT LEAST ONE HYDROPHOBIC SILICA

(30) Priorité: 01.06.2012 FR 1255113; 01.06.2012 US 201261654675 P
(43) Date de publication de la demande: 08.04.2015
(73) Titulaire: Galderma Research & Development, 06410 Biot (FR)
(72) Inventeur: DUPRAT, Agnès, F-06250 Mougins (FR)
(74) Mandataire: Casalonga
(86) Numéro de dépôt international: PCT/EP2013/061185
(87) Numéro de publication internationale: WO 2013/178745

(56) Documents cités:
- WO-A1-2006/066978
- DE-A1- 19 946 184

## Description

La présente invention porte sur des compositions topiques de type gel aqueux sous forme de suspension homogène d'un principe actif de la classe des rétinoïdes contenant au moins une silice hydrophobe, sur leur mode de préparation et sur leur utilisation dans le traitement de pathologies dermatologiques.

Les rétinoïdes sont utilisés dans le traitement de nombreuses pathologies dermatologiques et se révèlent notamment efficaces dans le traitement de dermatoses comme l'acné et le psoriasis. Cependant, l'application topique de rétinoïdes peut entraîner une irritation de la peau, une sécheresse et un érythème.

L'utilisation d'une forme galénique sous forme de gel aqueux dans laquelle le rétinoïde se trouverait sous forme dispersée semblait être une voie intéressante pour essayer de minimiser l'irritation grâce à une libération lente et prolongée du rétinoïde à partir de sa forme dispersée, ce qui aurait pu conduire à une pénétration cutanée progressive.

Toutefois, la réalisation de ce type de suspension homogène dans les conditions habituelles de formulation a présenté de nombreux inconvénients, lesquels n'ont pas permis d'obtenir la suspension homogène attendue.

Habituellement, les suspensions, c'est-à-dire les dispersions d'un solide insoluble et finement divisé dans un milieu liquide, se font à l'aide d'agents mouillants ou dispersants et/ou d'agents non solvant du principe actif. Ces agents permettent de disperser de façon homogène les particules de principe actif et de les maintenir en suspension dans la composition pharmaceutique.

Or, l'utilisation d'un de ces agents seul, ou d'un mélange d'agent mouillant et de non solvant du rétinoïde utilisé, n'a pas permis d'obtenir une suspension homogène, c'est-à-dire une bonne dispersion des particules de rétinoïde dans le milieu liquide. Cette mise en oeuvre a plutôt conduit soit à une solubilisation partielle du rétinoïde, soit à la formation d'agglomérats, soit à une instabilité physique de la composition.

A titre d'exemple, le document DE 199 46 181 propose des émulsions comprenant une huile polaire liquide et un composé permettant d'augmenter la polarité de cette huile.

Il y avait donc un besoin de trouver un moyen technique de réaliser une telle suspension homogène d'un principe actif de la classe des rétinoïdes dans un gel aqueux et d'en évaluer la stabilité physique, la pénétration, l'efficacité et la tolérance.

Il a été trouvé, de façon inattendue, que l'utilisation d'une silice hydrophobe permettait de résoudre ce problème et d'obtenir des suspensions homogènes d'un principe actif de la classe des rétinoïdes dans un gel aqueux, présentant ainsi une bonne stabilité physique, une bonne stabilité chimique et une bonne tolérance cutanée.

La façon dont la silice hydrophobe est mise en oeuvre au cours du procédé de préparation de ces suspensions homogènes est également particulière et déterminante pour l'obtention du résultat recherché. Le procédé de préparation de ces compositions constitue donc une partie de l'invention. Cette invention, qui va maintenant être présentée ci-dessous, s'applique à tout principe actif de la classe des rétinoïdes, plus particulièrement à ceux décrits dans la demande WO 2006/0066978 et encore plus particulièrement à l'acide 3"-*tert*-butyl-4'-(2-hydroxy-ethoxy)-4"-pyrrolidin-1-yl-[1,1';3',1"]-terphenyl-4-carboxylique.

### Résumé de l'invention

Un premier objet selon l'invention concerne une composition pharmaceutique comprenant
a) un principe actif de la classe des rétinoïdes,
b) au moins un agent gélifiant,
c) au moins une silice hydrophobe, et
d) de l'eau

De manière avantageuse, la composition selon l'invention peut également comprendre au moins un agent conservateur.

Un second objet selon l'invention concerne un procédé de préparation d'une composition pharmaceutique telle que décrite précédemment et comprenant les étapes suivantes :
a) une première étape d'ajout de 20% à 80% en poids de la totalité de l'agent gélifiant dans la quantité totale d'eau,,
b) une seconde étape de pré-mélange à l'état solide du principe actif de la classe des rétinoïdes avec au moins une silice hydrophobe,
c) une troisième étape, à température ambiante et sous homogénéisation, d'ajout du pré-mélange obtenu à l'étape b) sur le gel liquide obtenu à l'étape a), et enfin
d)une quatrième étape d'addition sous homogénéisation de la quantité restante d'agent gélifiant sur le milieu obtenu à l'étape c).

Lorsque la composition contient également un ou plusieurs agents conservateurs, ceux-ci sont avantageusement solubilisés dans l'eau avant l'étape a) d'ajout d'une partie de l'agent gélifiant.

Un troisième objet selon l'invention concerne une composition pharmaceutique telle que décrite ci-dessus pour son utilisation en tant que médicament.

Un quatrième objet selon l'invention concerne une composition pharmaceutique telle que décrite ci-dessus pour son utilisation dans le traitement de pathologies dermatologiques liées à un désordre de la kératinisation portant sur la différenciation ou sur la prolifération cellulaire.

Un cinquième objet selon l'invention concerne une composition pharmaceutique telle que décrite ci-dessus pour son utilisation dans le traitement de pathologies dermatologiques telles que définies ci-dessus et choisies dans le groupe comprenant
1) les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) les troubles de la kératinisation, notamment les ichtyoses, les états ichtyosiformes, l'ichtyose lamellaire, la maladie de Darrier, les kératodermies palmoplantaires, les leucoplasies, le pityriasis rubra pilaire et les états leucoplasiformes, le lichen cutané ou muqueux (buccal) ;
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczema;
4) les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose, les pigmentations et les rides ;
5) Toute condition liée à des proliférations dermiques ou épidermiques bénignes, qu'elles soient ou non d'origine virale telles que verrues vulgaires, les verrues planes , le molluscum contagiosum et l'épidermodysplasie verruciforme, les papillomatoses orales ou florides,;
6) les désordres dermatologiques tels que les dermatoses immunes comme le lupus érythémateux, les maladies immunes bulleuses et les maladies du collagène, telle la sclérodermie ;
7) les stigmates de l'atrophie épidermique et/ou dermique induite par les corticostéroïdes locaux ou systémiques, ou toute autre forme d'atrophie cutanée,
8) les troubles de la cicatrisation, ou pour prévenir ou pour réparer les vergetures, ou encore pour favoriser la cicatrisation,
9) dans le traitement de toute affection d'origine fongique au niveau cutané tel que le tinea pedis et le tinea versicolor,
10) les désordres de la pigmentation, tel l'hyperpigmentation, le mélasma, l'hypopigmentation ou le vitiligo ;
11) les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tel que le lymphome T.

L'invention sera décrite plus en détail dans la description et les exemples qui suivent, ainsi que dans les figures annexées dans lesquelles :

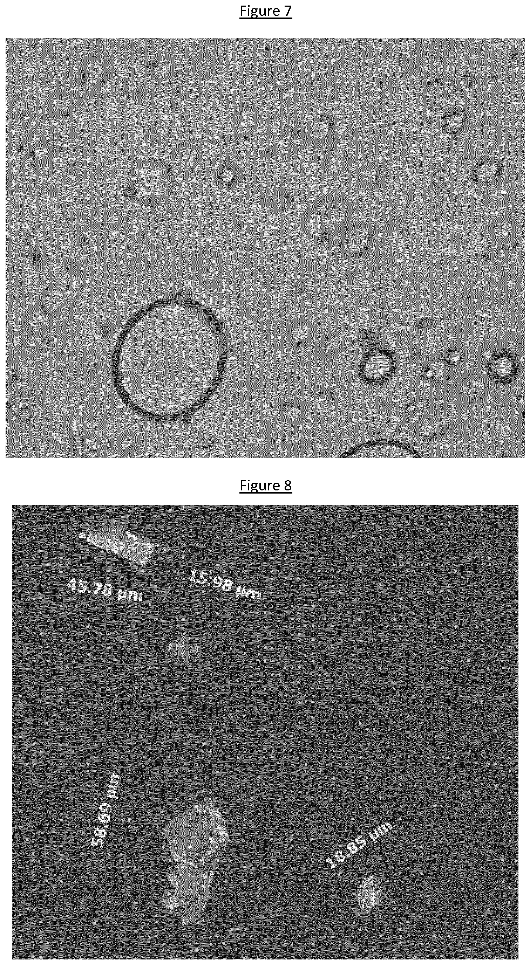

Les Figures 1 et 7 montrent les résultats d'essais de dispersion directe de l'acide 3"-tert-butyl-4'-(2-hydroxy-ethoxy)-4"-pyrrolidin-1-yl-[1,1';3',1"]-terphenyl-4-carboxylique dans des non-solvants.
Les Figures 3, 8 et 9 montrent les résultats d'essais de dispersion directe de l'acide 3"-tert-butyl-4'-(2-hydroxy-ethoxy)-4"-pyrrolidin-1-yl-[1,1';3',1"]-terphenyl-4-carboxylique dans des agents mouillants.

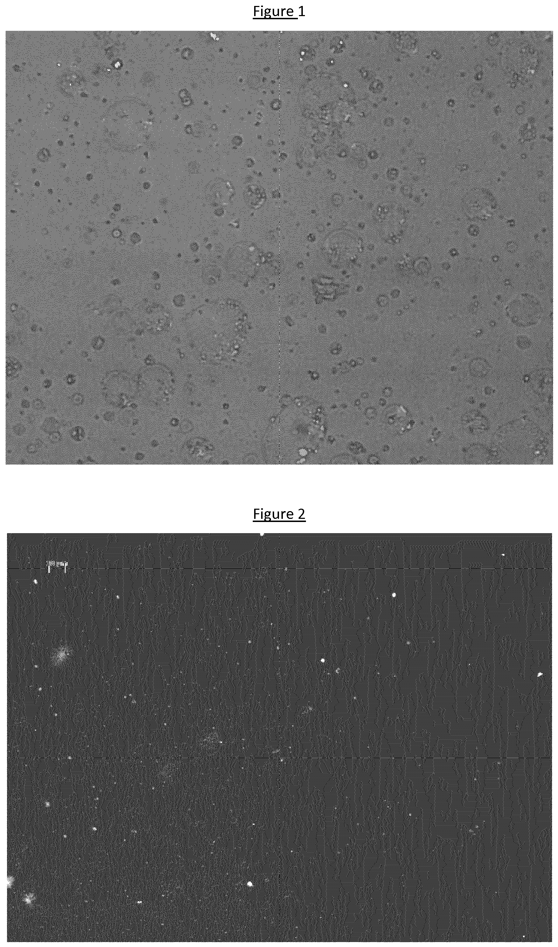

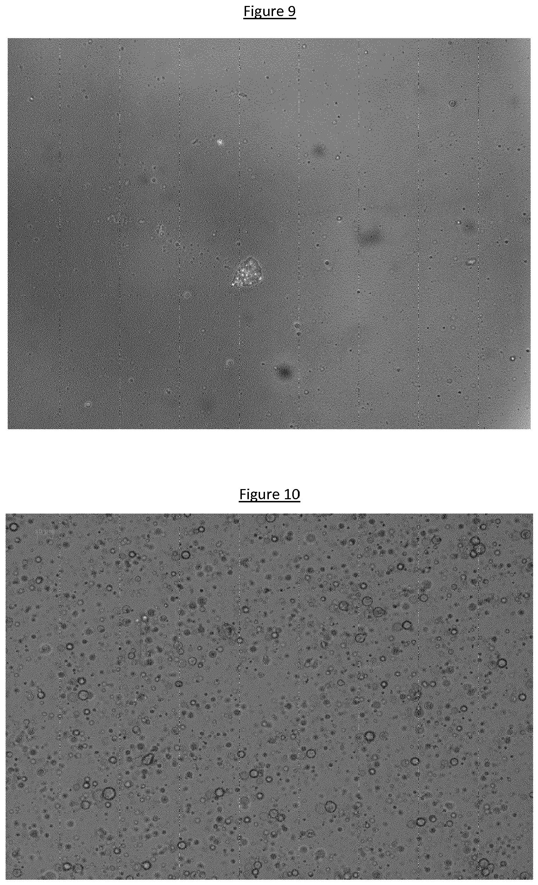

Les Figures 2, 4 et 10 montrent l'aspect microscopique de compositions selon l'invention.

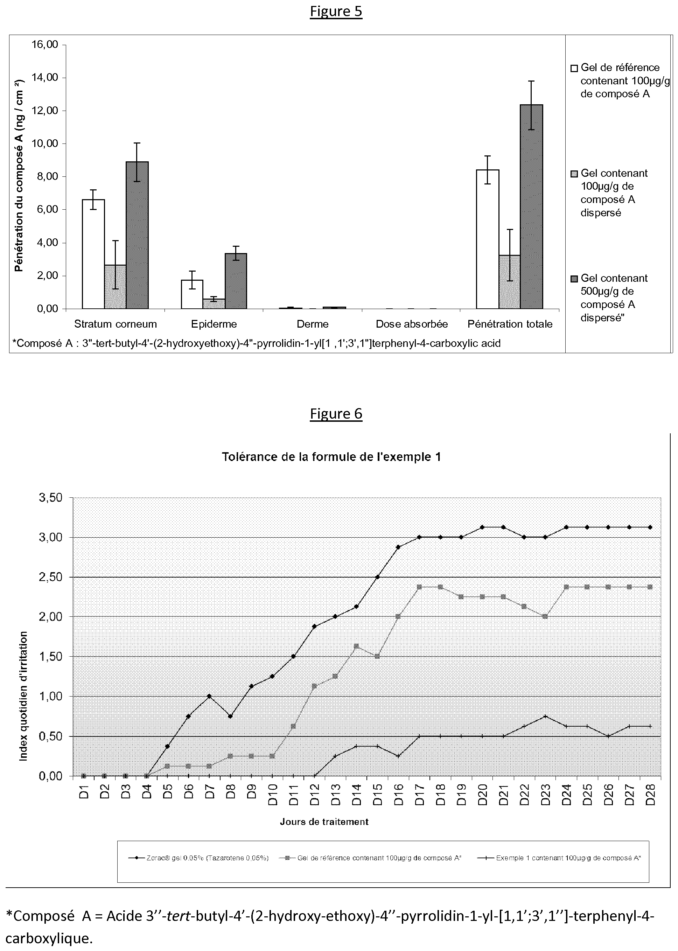

La Figure 5 illustre les profils de distribution dans les différents compartiments de la peau de gels selon l'invention par rapport à un gel de référence.
La Figure 6 illustre le profil de tolérance d'une composition selon l'invention par rapport à un gel de référence et à une composition comparative.

### Description détaillée de l'invention

La demanderesse a ainsi découvert de façon surprenante que l'utilisation d'une quantité efficace de silice hydrophobe permettait d'obtenir une suspension homogène d'un principe actif de la classe des rétinoïdes dans un milieu liquide sous forme de gel aqueux en l'absence d'agents mouillants ou dispersants.

Par gel aqueux, on entend une forme galénique semi-solide qui contient un agent gélifiant permettant de conférer une consistance à une solution ou une dispersion colloïdale hydrophile. Un gel peut contenir des particules en suspension.

Par suspension, on entend la dispersion d'un solide insoluble, ou quasiment insoluble, et finement divisé (i.e. le principe actif de la classe des rétinoïdes) dans un milieu liquide ou semi-solide. C'est par conséquent un dispositif hétérogène constitué d'une phase continue externe liquide et d'une phase interne solide sous forme de particules.

Habituellement, les dispersions de principes actifs se font à l'aide d'agents mouillants, d'agents dispersants ou à l'aide d'agents non solvant du principe actif. Ces agents permettent habituellement de disperser de façon homogène les particules de principe actif et de les maintenir en suspension dans la composition pharmaceutique.

Contrairement au résultat attendu par l'homme du métier, l'utilisation de ces agents, seuls ou en combinaison, n'a pas permis d'obtenir une suspension homogène d'un principe actif de la classe des rétinoïdes dans un milieu liquide sous forme de gel aqueux.

A titre d'illustration, l'utilisation du Marcol152 comme agent non solvant (lipophile) de l'acide 3"-*tert*-butyl-4'-(2-hydroxy-ethoxy)-4"-pyrrolidin-1-yl-[1,1';3',1"]-terphenyl-4-carboxylique pris à titre d'exemple de principe actif de la classe des rétinoïdes n'a pas permis d'obtenir une suspension homogène dans le gel aqueux décrit en tableau 1.

**Tableau 1:**

| **DENOMINATION COMMERCIALE** | **DENOMINATION INCI** | **%** |
|---|---|---|
| Acide 3"-*tert*-butyl-4'-(2-hydroxy-ethoxy)-4"-pyrrolidin-1-yl-[1,1';3',1"]-terphenyl-4-carboxylique micronisé | Acide 3"-*tert*-butyl-4'-(2-hydroxy-ethoxy)-4"-pyrrolidin-1-yl-[1,1';3',1"]-terphenyl-4-carboxylique micronisé | 0,01 |
| MARCOL 152 | Mineral oil | 0,20 |
| ACIDE BENZOIQUE | Benzoic acid | 0,10 |
| SORBATE DE POTASSIUM | Potassium Sorbate | 0,10 |
| SIMULGEL 600 PHA | Acrylamide, AMPS Copolymer Dispersion 40%/ Isohexadecane / Polysorbate 80 | 3,00 |
| EAU PURIFIEE | Purified water | QSP 100.00 |

Ainsi, dans cet exemple, la composition obtenue ne se présente pas sous forme d'une suspension homogène, mais présente plutôt des agglomérats de l'acide 3"-*tert*-butyl-4'-(2-hydroxy-ethoxy)-4"-pyrrolidin-1-yl-[1,1';3',1"]-terphenyl-4-carboxylique ayant une taille comprise entre 10 et 30 µm, Ces agglomérats sont localisés à la surface des gouttes de non solvant (lipophile) tel qu'illustré sur la Figure 1.

De façon surprenante, l'ajout, dans la composition du tableau 1, d'Aerosil® R972 comme silice hydrophobe, a permis d'obtenir une dispersion homogène dans le gel aqueux décrit en tableau 2.

**Tableau 2:**

| **DENOMINATION COMMERCIALE** | **DENOMINATION INCI** | **%** |
|---|---|---|
| Acide 3"-*tert*-butyl-4'-(2-hydroxy-ethoxy)-4"-pyrrolidin-1-yl-[1,1';3',1"]-terphenyl-4-carboxylique micronisé | Acide 3"-*tert*-butyl-4'-(2-hydroxy-ethoxy)-4"-pyrrolidin-1-yl-[1,1';3',1"]-terphenyl-4-carboxylique micronisé | 0,01 |
| Aérosil® R972 | Colloïdal anhydrous silica | 0,0025 |
| MARCOL 152 | Mineral oil | 0,20 |
| ACIDE BENZOIQUE | Benzoic acid | 0,08 |
| SORBATE DE POTASSIUM | Potassium Sorbate | 0,08 |
| SIMULGEL 600 PHA | Acrylamide, AMPS Copolymer Dispersion 40%/ Isohexadecane / Polysorbate 80 | 3,00 |
| EAU PURIFIEE | Purified water | QSP 100.00 |

Dans ces conditions, la composition obtenue présente maintenant une taille de particules d'acide 3"-*tert*-butyl-4'-(2-hydroxy-ethoxy)-4"-pyrrolidin-1-yl-[1,1';3',1"]-terphenyl-4-carboxylique inférieure à celle obtenue précédemment, comprise entre 2 et 10 µm, et sous forme d'une suspension homogène tel qu'illustré sur la Figure 2.

De même, à titre d'illustration, l'utilisation de PLURONIC L44, comme agent mouillant ou dispersant, en association avec de la GLYCERINE et du ST-CYCLOMETHICONE-5, comme agent non solvant de l'acide 3"-*tert*-butyl-4'-(2-hydroxy-ethoxy)-4"-pyrrolidin-1-yl-[1,1';3',1"]-terphenyl-4-carboxylique pris à titre d'exemple de principe actif de la classe des rétinoïdes n'a pas permis d'obtenir une suspension homogène dans le gel aqueux décrit en tableau 3.

**Tableau 3:**

| **DENOMINATION COMMERCIALE** | **DENOMINATION INCI** | **%** |
|---|---|---|
| Acide 3"-*tert*-butyl-4'-(2-hydroxy-ethoxy)-4"-pyrrolidin-1-yl-[1,1';3',1"]-terphenyl-4-carboxylique micronisé | Acide 3"-*tert*-butyl-4'-(2-hydroxy-ethoxy)-4"-pyrrolidin-1-yl-[1,1';3',1"]-terphenyl-4-carboxylique micronisé | 0,01 |
| PLURONIC L44 | Poloxamer 124 | 0,10 |
| GLYCERINE | Glycerin | 1,00 |
| ST-CYCLOMETHICONE-5 | Cyclopentasiloxane | 2,00 |
| RONACARE ALLANTOINE | Allantoin | 0,20 |
| SIMULGEL 600 PHA | Acrylamide, AMPS Copolymer Dispersion 40%/ Isohexadecane / Polysorbate 80 | 2,00 |
| EAU PURIFIEE | Purified water | QSP 100.00 |

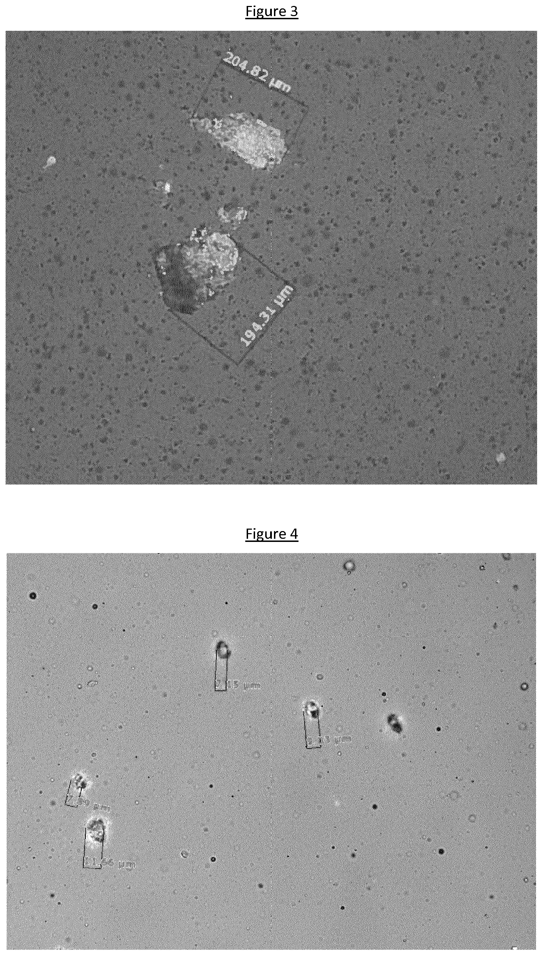

Ainsi, dans cet exemple, la composition obtenue ne se présente pas sous forme d'une suspension homogène, mais présente plutôt des agglomérats de l'acide 3"-*tert*-butyl-4'-(2-hydroxy-ethoxy)-4"-pyrrolidin-1-yl-[1,1';3',1"]-terphenyl-4-carboxylique ayant une taille comprise entre 10 et 250 µm tel qu'illustré sur la Figure 3.

De façon surprenante, l'ajout dans la composition du tableau 1 d'Aérosil® R972, comme silice hydrophobe, à la place de la combinaison agent dispersant/agents non solvant, a permis d'obtenir une suspension homogène dans le gel aqueux décrit en tableau 4.

**Tableau 4:**

| **DENOMINATION COMMERCIALE** | **DENOMINATION INCI** | % |
|---|---|---|
| Acide 3"-*tert*-butyl-4'-(2-hydroxy-ethoxy)-4"-pyrrolidin-1-yl-[1,1';3',1"]-terphenyl-4-carboxylique micronisé | Acide 3"-*tert*-butyl-4'-(2-hydroxy-ethoxy)-4"-pyrrolidin-1-yl-[1,1';3',1"]-terphenyl-4-carboxylique micronisé | 0,008 |
| Aérosil® R972 | Colloïdal anhydrous silica | 0,002 |
| ACIDE BENZOIQUE | Benzoic acid | 0,06 |
| SORBATE DE POTASSIUM | Potassium Sorbate | 0,06 |
| SIMULGEL 600 PHA | Acrylamide, AMPS Copolymer Dispersion 40%/ Isohexadecane / Polysorbate 80 | 3,00 |
| EAU PURIFIEE | Purified water | QSP 100.00 |

Dans ces conditions, la composition obtenue présente maintenant une taille de particules d'acide 3"-*tert*-butyl-4'-(2-hydroxy-ethoxy)-4"-pyrrolidin-1-yl-[1,1';3',1"]-terphenyl-4-carboxylique inférieure à celle obtenue précédemment, comprise entre 2 et 10 µm, et sous forme d'une suspension homogène tel qu'illustré sur la Figure 4.

Ainsi, un premier objet selon l'invention concerne donc une composition pharmaceutique comprenant :
a) un principe actif de la classe des rétinoïdes,
b) au moins un agent gélifiant,
c) au moins une silice hydrophobe, et
d) de l'eau

Un mode particulier selon le premier objet de l'invention concerne une composition pharmaceutique comprenant :
a) un principe actif de la classe des rétinoïdes,
b) au moins un non solvant lipophile du principe actif,
c) au moins un agent gélifiant,
d) au moins une silice hydrophobe, et
e) de l'eau
Cette composition peut également comprendre au moins un agent conservateur.

### Principe actif de la classe des rétinoïdes

Par principe actif de la classe des rétinoïdes, on entend tout type de rétinoïde. A titre d'exemple de rétinoïde, on peut citer la Trétinoïne, aussi appelée acide rétinoïque, ou acide tout trans rétinoïque, l'Isotrétinoïne, aussi appelée acide 13-cis rétinoïque, l'Adapalène, le Tazarotène, le Rétinol, le Retinaldehyde ou encore tout rétinoïde tel que décrit dans la demande WO 2006/0066978.

Parmi ces composés, on préfère utiliser ceux décrits dans la demande WO 2006/0066978 et représentés par la formule générale (I) suivante : 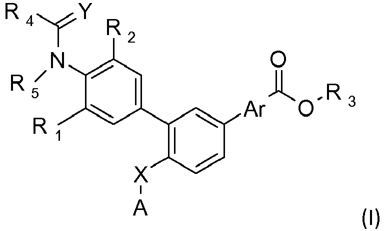 dans laquelle :
- R₁ est un atome d'hydrogène, un radical alkyle de 1 à 4 atomes de carbone ou un radical-CF₃;
- R₂ est un atome d'hydrogène, un radical alkyle ou alkoxy de 1 à 4 atomes de carbone ou un atome de chlore ;
- R₃ est un atome d'hydrogène, un radical alkyle ou alkoxy de 1 à 10 atomes de carbone et de préférence 1 à 6 atomes de carbone, linéaire ou ramifié éventuellement substitué par un groupement méthoxy, ou encore un radical alkyle de 1 à 10 atomes de carbone et de préférence 1 à 6 atomes de carbone, linéaire ou ramifié, contenant une fonction éther ;
- R₄ est un atome d'hydrogène, un radical alkyle de 1 à 3 atomes de carbone ;
- R₅ est un atome d'hydrogène, un radical alkyle de 1 à 3 atomes de carbone ;
- ou bien R₄ et R₅ forment ensemble avec la liaison -N-C(=Y)- un cycle de type pyrrolidine, pyrrolidinone, piperidine ou piperidinone ;
- Y représente deux atomes d'hydrogène ou un hétéroatome comme l'oxygène, le soufre ;
- Ar représente un cycle 1,4-phényl, 2,5-pyridyl, 5,2-pyridyl ou 2,5-thiophényl ;
- X représente un atome d'oxygène éventuellement substitué par une chaîne alkyl ou alkylamine ou une liaison simple C-C;
- A représente un atome d'hydrogène ou la formule suivante : 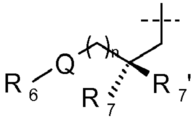 dans laquelle,
   ▪ Q est un atome d'oxygène ou la liaison -NH- ;
   ▪ R₆ représente un atome d'hydrogène, un radical alkyle de 1 à 6 atomes de carbone, un radical cycloalkyle de 3 à 6 atomes de carbone, un radical -C(O)CH3 ou-C(O)CH2CH3 ;
   ▪ R₇ et R₇' représentent, indépendamment l'un de l'autre, un atome d'hydrogène ou un groupement hydroxyle, à la condition que R₇ et R₇' ne soient pas simultanément un groupement hydroxyle ;
   ▪ n vaut 0, 1, 2 , 3, 4 ou 5 ;
et les sels des composés de formule (I) lorsque R₃ représente un atome d'hydrogène ainsi que les isomères géométriques desdits composés de formule (I).

Parmi ces composés, on préfère utiliser l'acide 3"-*tert*-butyl-4'-(2-hydroxy-ethoxy)-4"-pyrrolidin-1-yl-[1,1';3',1"]-terphenyl-4-carboxylique dans les compositions de la présente invention.

Dans les compositions de la présente invention, le principe actif de la classe des rétinoïdes est de préférence utilisé à des concentrations allant de 0.00001% à 5% en poids par rapport au poids total de la composition et préférentiellement de 0.005% à 1% en poids.

### Agent non solvant lipophile

Dans l'invention, les compositions peuvent contenir un ou plusieurs non solvants lipophiles du principe actif de la classe des rétinoïdes.

Le ou les solvants lipophiles peuvent être choisis parmi les huiles minérales liquides comme par exemple les produits Marcol 152 ou Primol 352, le perhydrosqualène par exemple vendu sous le nom de Cosbiol, les Cyclomethicones comme par exemple le ST Cyclomethicone 5, le Q7 79120 Silicone fluid 100 cst, les dimethicones comme par exemple le Fluide DC 225 10cst.

Le ou les non solvants lipophiles sont de préférence utilisés à des concentrations allant de 0.1% à 20% en poids par rapport au poids total de la composition et préférentiellement de 0.1% à 5%. En poids

### Agent gélifiant

Par agent gélifiant, on entend un composé polymère apte à conférer à la composition la texture d'un gel.

Le ou les agents gélifiants peuvent être notamment choisis parmi les polymères d'origine végétale, les gommes, les pectines, la cellulose et ses dérivés, les polymères d'origine microbiologiques tel que la gomme xanthane, et les polymères gélifiants d'origine synthétique.

L'agent gélifiant est plus préférentiellement choisi dans la liste comprenant :
- les « Acrylates/C10-30 Alkyl Acrylate Crosspolymers » vendus sous le nom de Pemulen TR-1 ou Pemulen TR-2 par la société Lubrizol,
- les gélifiants de la famille des polyacrylamides comme le mélange Sodium acrylamide/ acryloyldimethyltaurate copolymer / isohexadecane / polysorbate 80 vendu sous le nom de Simulgel 600PHA par la société SEPPIC,
- le mélange polyacrylamide/isoparaffine C13-14/laureth-7 vendu sous le nom de Sepigel 305 par la société SEPPIC,
- les carbomers vendus sous le nom d'Ultrez 20®, d'Ultrez 10®, de Carbopol 1382® ou de Carbopol ETD2020NF®, de carbopol 981 ou encore carbopol 980 par la Société Lubrizol,
- les polysaccharides avec à titre d'exemples non limitatifs la gomme de xanthane telle que le Xanturall80® vendu par la société Kelco, la gellan gum vendu sous le nom de Kelcogel par la société Kelco, la gomme guar, la cellulose et ses dérivés tel que la microcristalline cellulose et carboxymethyl cellulose de sodium vendue sous le nom d'Avicel CL-611 par la société FMC Biopolymer, l'hydroxypropylmethylcellulose en particulier le produit vendu sous le nom de Methocel E4M premium par la société Dow Chemical ou l'hydroxyéthylcellulose , en particulier, le produit vendu sous le nom de Natrosol HHX 250® par la société Ashland, la sodium carboxymethylcellulose en particulier la Blanose cellulose gum 7F vendu par la société Ashland,
- la famille des aluminium magnésium silicates tel que le Veegum K vendu par la société Vanderbilt,
- la famille des polymères acryliques couplés à des chaînes hydrophobes tel que le PEG-150/decyl/SMDI copolymer vendu sous le nom de Aculyn 44 (polycondensat comprenant au moins comme éléments, un polyéthylèneglycol à 150 ou 180 moles d'oxyde d'éthylène, de l'alcool décylique et du méthylène bis(4-cyclohexylisocyanate) (SMDI), à 35% en poids dans un mélange de propylèneglycol (39%) et d'eau (26%)),
- la famille des amidons modifiés tels que l'amidon de pomme de terre modifié vendu sous le nom de Structure Solanace ou bien leurs mélanges,
- la famille des carraghénanes en particulier réparties sous quatre grandes familles : κ, λ, β, ω tel que les Viscarin® et les Gelcarin® commercialisés par la société IMCD.

De façon préférentielle, un gélifiant de type polyacrylamide sera utilisé. Parmi les gélifiants de ce type, le Simulgel 600 PHA®, le Sepigel 305 ou encore les Pemulen TR1 et TR2, connus pour leurs propriétés épaississantes et stabilisantes, seront préférés.

Le ou les gélifiants entrant dans la composition selon l'invention sont de préférence utilisés à des concentrations allant de 0,005% à 5 % en poids par rapport au poids total de la composition et préférentiellement, allant de 1% à 4% en poids ;.

### Agent conservateur

Par agent conservateur, on entend toute substance capable de s'opposer aux altérations d'origine chimique ou microbiologique d'un produit.

Dans l'invention, les compositions peuvent comprendre un ou plusieurs agents conservateurs, tels le méthyl parabène, le propyl parabène, le chlorure de benzalkonium, le phénoxyéthanol, l'alcool benzylique, le sorbate de potassium, l'acide benzoïque, le 2-Bromo-2-Nitropropane-1,3-Diol ou Bronopol, la chlorhexidine, le chlorocrésol et ses dérivés, le sodium benzoate, l'alcool éthylique et la diazolidinylurée. Ces conservateurs peuvent être utilisés seul ou en association afin de protéger efficacement les formules contre toute contamination bactérienne.

Ils sont utilisés à des concentrations préférentielles allant de 0.01% à 5% en poids par rapport au poids total de la composition et plus préférentiellement de 0.01 à 2% en poids.

### Silice hydrophobe

La composition contient une ou plusieurs silices hydrophobes. La quantité de silice(s) hydrophobe(s) est de préférence comprise entre 0,0001% et 5% en poids par rapport au poids total de la composition et plus préférentiellement, entre 0,0001% et 1% en poids.Les silices hydrophobes utilisées dans la composition de l'invention sont de préférence amorphes et d'origine pyrogénée. Elles se présentent de préférence sous forme pulvérulente.

Avantageusement selon la présente invention, la silice hydrophobe a une surface spécifique (mesurée par la méthode BET) supérieure ou égale à 100 m²/g, de manière encore plus avantageuse supérieure ou égale à 150 m²/g, de manière encore plus avantageuse supérieure ou égale à 190 m²/g, de manière encore plus avantageuse supérieure ou égale à 220 m²/g, et encore plus avantageusement supérieure ou égale à 250 m²/g. La silice hydrophobe selon la présente invention a avantageusement un revêtement à base d'hydrocarbure ou de silicone. De préférence, la silice hydrophobe est revêtue d'un revêtement à base de silicone, par exemple d'organosiloxane, de polysiloxane, ou d'huile de silicone. A titre d'exemple, des silices telles que les silices triméthylsiloxylée, diméthylsiloxylée, octylsiloxylée et les silices revêtues d'huile de silicone peuvent être utilisées.

Dans un mode de réalisation particulier de la présente invention, la silice hydrophobe a une surface spécifique (mesurée par la méthode BET) de 110 à 330 m²/g, avantageusement de 110 à 260 m²/g, avantageusement de 115 à 220 m²/g, avantageusement de 115 à 190 m²/g, avantageusement de 115 à 150 m²/g, encore avantageusement de 115 à 140 m²/g, encore avantageusement d'environ 130 m²/g, et une grosseur moyenne de particules primaires inférieure à 30 nm, avantageusement entre 5 et 20 nm.

La silice hydrophobe est choisie dans la liste comprenant les silices Aérosil® R972, R974, R104, R106, R202, R805, R812, R812S, R816, R7200, R8200, R9200 et R711 vendues par Evonik et telles que décrites dans le tableau 5 ci-dessous, les silices pyrogéniques hydrophobes HDK 13L et H2000 vendues par Wacker et les mélanges de ces différentes silices.

**Tableau 5: Silices hydrophobes**

| **Grades d'AEROSIL®** | **BET surface spécifique [m²/g]** | **pH** | **Teneur en carbone [% en poids]** |
|---|---|---|---|
| AEROSIL® R972 | 110 ± 20 | 3.6-5.5 | 0.6-1.2 |
| AEROSIL® R974 | 170 ± 20 | 3.7-4.7 | 0.7-1.3 |
| AEROSIL® R104 | 150 ± 25 | ≥ 4.0 | 1.0-2.0 |
| AEROSIL® R106 | 250 ± 30 | ≥ 3.7 | 1.5-3.0 |
| AEROSIL® R202 | 100 ± 20 | 4.0-6.0 | 3.5-5.0 |
| AEROSIL® R805 | 150 ± 25 | 3.5-5.5 | 4.5-6.5 |
| AEROSIL® R812 | 260 ± 30 | 5.5-7.5 | 2.0-3.0 |
| AEROSIL® R812S | 220 ± 25 | 5.5-7.5 | 3.0-4.0 |
| AEROSIL® R816 | 190 ± 20 | 4.0-5.5 | 0.9-1.8 |
| AEROSIL® R7200 | 150 ± 25 | 4.0-6.0 | 4.5-6.5 |
| AEROSIL® R8200 | 160 ± 25 | ≥ 5.0 | 2.0-4.0 |
| AEROSIL® R9200 | 170 ± 20 | 3.4-5.0 | 0.7-1.3 |
| AEROSIL® R711 | 150 ± 25 | 4.0-6.0 | 4.5-6.5 |

En particulier, la silice préférée est une silice Aérosil® R972 produite par EVONIK. Pour le type Aérosil® R972, il sera préféré un grade Pharma qui est une silice colloïdale hydrophobe (INCI : Silica Dimethyl Silylate) d'une grande pureté, amorphe, anhydre, pour une utilisation comme excipient dans les produits pharmaceutiques (grade testé selon la Pharmacopée Européenne et selon la monographie de l'USP / NF).

L'Aérosil® R972 est une silice hydrophobe obtenue après traitement d'une silice hydrophile avec du DDS (Dimethyl Dichloro Silane).

Selon un mode particulier, le premier objet selon l'invention concerne une composition pharmaceutique comprenant :
a) un principe actif de la classe des rétinoïdes représenté par la formule générale(I) 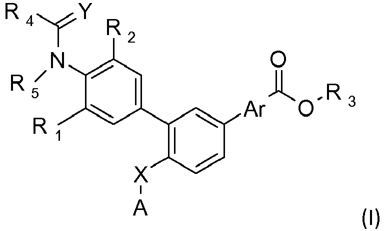 dans laquelle :
   - R₁ est un atome d'hydrogène, un radical alkyle de 1 à 4 atomes de carbone ou un radical-CF₃;
   - R₂ est un atome d'hydrogène, un radical alkyle ou alkoxy de 1 à 4 atomes de carbone ou un atome de chlore ;
   - R₃ est un atome d'hydrogène, un radical alkyle ou alkoxy de 1 à 10 atomes de carbone et de préférence 1 à 6 atomes de carbone, linéaire ou ramifié éventuellement substitué par un groupement méthoxy, ou encore un radical alkyle de 1 à 10 atomes de carbone et de préférence 1 à 6 atomes de carbone, linéaire ou ramifié, contenant une fonction éther ;
   - R₄ est un atome d'hydrogène, un radical alkyle de 1 à 3 atomes de carbone ;
   - R₅ est un atome d'hydrogène, un radical alkyle de 1 à 3 atomes de carbone ;
   - ou bien R₄ et R₅ forment ensemble avec la liaison -N-C(=Y)- un cycle de type pyrrolidine, pyrrolidinone, piperidine ou piperidinone ;
   - Y représente deux atomes d'hydrogène ou un hétéroatome comme l'oxygène, le soufre ;
   - Ar représente un cycle 1,4-phényl, 2,5-pyridyl, 5,2-pyridyl ou 2,5-thiophényl ;
   - X représente un atome d'oxygène éventuellement substitué par une chaîne alkyl ou alkylamine ou une liaison simple C-C;
   - A représente un atome d'hydrogène ou la formule suivante : 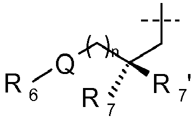 dans laquelle,
      ▪ Q est un atome d'oxygène ou la liaison -NH-;
      ▪ R₆ représente un atome d'hydrogène, un radical alkyle de 1 à 6 atomes de carbone, un radical cycloalkyle de 3 à 6 atomes de carbone, un radical -C(O)CH3 ou-C(O)CH2CH3 ;
      ▪ R₇ et R₇' représentent, indépendamment l'un de l'autre, un atome d'hydrogène ou un groupement hydroxyle, à la condition que R₇ et R₇' ne soient pas simultanément un groupement hydroxyle ;
      ▪ n vaut 0, 1, 2 , 3, 4 ou 5 ;
   et les sels des composés de formule (I) lorsque R₃ représente un atome d'hydrogène ainsi que les isomères géométriques desdits composés de formule (I),
b) au moins un agent gélifiant,
c) au moins une silice hydrophobe, et
d) de l'eau

Selon un mode particulier, le premier objet selon l'invention concerne une composition pharmaceutique comprenant :
a) l'acide 3"-*tert*-butyl-4'-(2-hydroxy-ethoxy)-4"-pyrrolidin-1-yl-[1,1';3',1"]-terphenyl-4-carboxylique,
b) au moins un agent gélifiant,
c) au moins une silice hydrophobe, et
d) de l'eau

Selon un mode particulier, le premier objet selon l'invention concerne une composition pharmaceutique comprenant :
a) de 0.00001% à 5% d'un principe actif de la classe des rétinoïdes,
b) de 0,005% à 5 % d'au moins un agent gélifiant,
c) de 0,0001% et 5% d'au moins une silice hydrophobe, et
d) de l'eau en quantité suffisante pour atteindre 100% du poids total de la composition.

Cette composition peut également comprendre de 0.01% à 5% d'au moins un agent conservateur.

Selon un mode plus particulier, le premier objet selon l'invention concerne une composition pharmaceutique comprenant :
a) de 0.0001% à 5% d'un principe actif de la classe des rétinoïdes,
b) de 0,1% à 20% d'au moins un non solvant lipophile du principe actif,
c) de 0,005% à 5 % d'au moins un agent gélifiant,
d) de 0,0001% et 5% d'au moins une silice hydrophobe, et
e) de l'eau en quantité suffisante pour atteindre 100% du poids total de la composition.

Cette composition peut également comprendre de 0.01% à 5% d'au moins un agent conservateur.

Selon un mode encore plus particulier, le premier objet selon l'invention concerne une composition pharmaceutique comprenant :
a) de 0.00001% à 5% d'acide 3"-*tert*-butyl-4'-(2-hydroxy-ethoxy)-4"-pyrrolidin-1-yl-[1,1';3',1"]-terphenyl-4-carboxylique,
b) de 0,005% à 5 % d'au moins un agent gélifiant,
c) de 0,0001% et 5% d'au moins une silice hydrophobe, et
d) de l'eau en quantité suffisante pour atteindre 100% du poids total de la composition.

Cette composition peut également comprendre de 0.01% à 5% d'au moins un agent conservateur.

Les compositions selon l'invention peuvent également contenir d'autres excipients comme par exemple :

### Des agents humectant

Les agents humectant sont choisis de préférence dans la liste comprenant la glycérine, le pentylène glycol, le dipropylène glycol, le propylène glycol, la diglycérine, le sorbitol et leurs mélanges. Leur quantité est comprise entre 1% et 40% en poids par rapport au poids total de la composition et préférentiellement entre 1% et 10%.

### Des agents chélatant

Les agents chélatant sont choisis de préférence dans la liste comprenant l'EDTA (ethylenediaminetetraacetic acid) et ses dérivés ou sels, la dihydroglycérine, les acides citriques et tartriques, la gluconolactone et leurs mélanges.

### Des antioxydants

Les antioxydants sont choisis de préférence dans la liste comprenant la vitamine E et ses dérivés, comme le DL alpha Tocophérol ou l'acétate de tocophérol, la vitamine C et ses dérivés, comme le Palmitate d'Ascorbyl, le Butylhydroxy toluène (Nipanox BHT) et leurs mélanges.

### Des agents apaisants et/ou anti-irritants

Les agents apaisants et/ou anti-irritants sont choisis de préférence dans la liste comprenant le PPG-12/SMDI copolymère (ou Polyolprepolymer-2), l'acide glycyrrhetinique ou ses dérivés comme par exemple l'Enoxolone, l'acide hyaluronique et ses sels comme par exemple le hyaluronate de sodium vendu sous le nom commercial de HYAL. NA PWD PH 15-51-45, l'allantoïne et leurs mélanges.

Les compositions selon l'invention présentent une bonne stabilité physique et une bonne stabilité chimique au cours du temps.

Une bonne stabilité physique correspond à un maintien de l'homogénéité de la phase dispersée au cours du temps (pas de sédimentation des particules, pas d'agglomérats, pas de dissolution des particules) ainsi qu'une bonne stabilité de l'aspect macroscopique et de la viscosité du produit au cours du temps.

Les limites fixées pour une bonne stabilité chimique du principe actif au cours du temps correspondent à un titre en principe actif compris entre 95 et 105% (le titre de l'actif est exprimé en % relatif par rapport au % initial effectué à T0).

Un second objet selon l'invention concerne un procédé de préparation d'une composition pharmaceutique telle que décrite précédemment et comprenant les étapes suivantes :
a) une première étape d'ajout de 20% à 80% en poids de la totalité de l'agent gélifiant dans la quantité totale d'eau,
b) une seconde étape de pré-mélange à l'état solide du principe actif de la classe des rétinoïdes avec au moins une silice hydrophobe,
c) une troisième étape, à température ambiante et sous homogénéisation, d'ajout du pré-mélange obtenu à l'étape b) au gel liquide obtenu à l'étape a), et enfin
d)une quatrième étape d'addition sous homogénéisation de la quantité restante d'agent gélifiant sur le milieu obtenu à l'étape c).

La première étape a) du procédé consiste à préparer un gel liquide en ajoutant à la totalité de l'eau purifiée entre 20% et 80% en poids de la totalité de l'agent gélifiant et préférentiellement entre 20% et 50% de façon à obtenir un gel liquide.

Lorsque la composition contient des agents conservateurs, ceux-ci sont solubilisés dans l'eau avant ajout du ou des agents gélifiants.

Dans la seconde étape b) du procédé, le principe actif de la classe des rétinoïdes est mélangé à l'état solide avec au moins une silice hydrophobe telle que décrite précédemment.

Optionnellement, le mélange solide obtenu peut être dispersé dans au moins un non solvant lipophile du principe actif de la classe des rétinoïdes.

Parmi les rétinoïdes mis en oeuvre à cette étape, on préférera utiliser ceux répondant à la formule générale (I) et préférentiellement l'acide 3"-*tert*-butyl-4'-(2-hydroxy-ethoxy)-4"-pyrrolidin-1-yl-[1,1';3',1"]-terphenyl-4-carboxylique.

Au cours de la troisième étape c), le mélange obtenu à l'étape b) est ajouté à température ambiante et sous homogénéisation au gel liquide obtenu à l'étape a).

Enfin, dans la dernière étape d), la quantité restante d'agent gélifiant est additionnée au milieu obtenu à l'étape c) afin d'obtenir une suspension homogène d'un principe actif de la classe des rétinoïdes dans un milieu liquide sous forme de gel aqueux.

Un mode particulier du second objet selon l'invention concerne donc un procédé de préparation d'une composition pharmaceutique telle que décrite précédemment et comprenant les étapes suivantes :
a) une première étape d'ajout de 20% à 80% en poids de la totalité de l'agent gélifiant dans la quantité totale d'eau,,
b) une seconde étape de pré-mélange à l'état solide du principe actif de la classe des rétinoïdes avec au moins une silice hydrophobe, suivie d'une dispersion du mélange solide obtenu dans au moins un non solvant lipophile du principe actif de la classe des rétinoïdes,
c) une troisième étape, à température ambiante et sous homogénéisation, d'ajout du pré-mélange obtenu à l'étape b) au gel liquide obtenu à l'étape a), et enfin
d)une quatrième étape d'addition sous homogénéisation de la quantité restante d'agent gélifiant sur le milieu obtenu à l'étape c).

Ainsi, les compositions pharmaceutiques selon l'invention sont préparées à partir d'un mélange du principe actif de la classe des rétinoïdes, de préférence sous forme micronisée, avec au moins une silice hydrophobe. Ce pré-mélange est ensuite, soit dispersé dans au moins un non solvant lipophile du principe actif de la classe des rétinoïdes pour obtenir une suspension homogène qui est ensuite ajoutée dans un gel aqueux, soit ce pré-mélange est ajouté directement dans un gel aqueux.

Les compositions pharmaceutiques selon l'invention sont conçues pour leur utilisation dans le traitement de pathologies dermatologiques liées à un désordre de la kératinisation portant sur la différenciation ou sur la prolifération cellulaire.

Ces compositions contiennent un principe actif de la classe des rétinoïdes.

Les rétinoïdes sont des ligands modulateurs des récepteurs nucléaires de l'acide rétinoïque (RARs). Les composés ayant une activité de type rétinoïde sont décrits dans la littérature comme ayant des activités dans les processus de prolifération et différentiation cellulaire. Ces propriétés confèrent à cette classe de composés, un fort potentiel dans le traitement ou la prévention de nombreuses pathologies, et plus particulièrement en dermatologie et dans le traitement des cancers.

Beaucoup d'effets biologiques des rétinoïdes sont dues à une modulation des RARs. Les rétinoïdes trouvent dans le cytosol des récepteurs protéiques spécifiques comme la *cellular retinoic acid-binding protein* (CRABP) et la *cellular retinol-binding protein* (CRBP). Ces deux protéines sont caractérisées par une spécificité et une affinité élevées pour leur ligand respectif, l'acide rétinoïque et le rétinol. Elles sont présentes dans un grand nombre de types cellulaires sans qu'existe de spécificité tissulaire. La CRBP et la CRABP joueraient un rôle important dans les effets cellulaires des rétinoïdes : il existe en particulier une corrélation entre leur présence et les tissus où les rétinoïdes exercent des effets biologiques. Au niveau cutané, le taux de CRABP est supérieur dans l'épiderme par rapport au derme, ce qui pourrait expliquer les importants effets épidermiques de l'acide rétinoïque.

L'acide rétinoïque se lie d'abord à la CRABP dans le cytosol. Le complexe CRABP- acide rétinoïque est transporté (pendant les seize premières heures in vitro) dans le noyau où l'acide rétinoïque est libéré. Il se fixe alors sur un autre récepteur protéique hautement spécifique, le récepteur nucléaire de l'acide rétinoïque (RAR), dont il existe au moins trois types (α, β et γ). La modulation de l'expression génomique se fait, au niveau de la peau, après complexation pour former un hétérodimère RAR-RXR (*retinoid X receptor*). Au niveau cutané, le RARγ forme 90% des RAR épidermiques, tandis que le RXR-α est prédominant.

Il y avait donc un besoin technique que les compositions selon l'invention permettent, après leur application topique, d'une part une libération du principe actif de la classe des rétinoïdes sous forme dispersée et d'autre part une pénétration ciblée dans la couche épidermique où se trouvent localisés les récepteurs nucléaires de l'acide rétinoïque (RARs).

De façon surprenante, il a été montré que les compositions de l'invention sous forme de suspension homogène d'un principe actif de la classe des rétinoïdes dans un gel aqueux pénètrent de façon ciblée dans l'épiderme tout en minimisant la pénétration dans le derme et par voie systémique, ce qui permet également de diminuer tout risque d'effets secondaires par voie systémique.

Cette pénétration ciblée est illustrée, à titre d'exemple, sur la Figure 5 (voir exemple 10) pour deux compositions sous forme de suspension de l'acide 3"-*tert*-butyl-4'-(2-hydroxy-ethoxy)-4"-pyrrolidin-1-yl-[1,1';3',1"]-terphenyl-4-carboxylique dans un gel aqueux.

Ainsi, dans les conditions utilisées et telles que décrites à l'exemple 10, le pourcentage d'acide rétinoïque ayant pénétré dans l'épiderme et le derme seize heure après application topique d'une composition selon l'invention représente entre 0,1% et 0,8% en poids de la dose appliquée, et de préférence entre 0,2% et 0,4% en poids de la dose appliquée.

De préférence, l'invention porte sur une composition pharmaceutique comprenant :
a) un principe actif de la classe des rétinoïdes,
b) au moins un agent gélifiant,
c) au moins une silice hydrophobe, et
d)de l'eau
dans laquelle la quantité de principe actif ayant pénétré dans l'épiderme et le derme 16 heures après application topique représente entre 0,1% et 0,8% en poids de la quantité appliquée et préférentiellement entre 0,2% et 0,4% en poids de la quantité appliquée.

De préférence, l'invention porte sur une composition pharmaceutique comprenant :
a) un principe actif de la classe des rétinoïdes représenté par la formule générale(I) 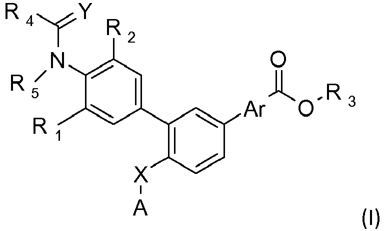 dans laquelle :
   - R₁ est un atome d'hydrogène, un radical alkyle de 1 à 4 atomes de carbone ou un radical-CF₃;
   - R₂ est un atome d'hydrogène, un radical alkyle ou alkoxy de 1 à 4 atomes de carbone ou un atome de chlore ;
   - R₃ est un atome d'hydrogène, un radical alkyle ou alkoxy de 1 à 10 atomes de carbone et de préférence 1 à 6 atomes de carbone, linéaire ou ramifié éventuellement substitué par un groupement méthoxy, ou encore un radical alkyle de 1 à 10 atomes de carbone et de préférence 1 à 6 atomes de carbone, linéaire ou ramifié, contenant une fonction éther ;
   - R₄ est un atome d'hydrogène, un radical alkyle de 1 à 3 atomes de carbone ;
   - R₅ est un atome d'hydrogène, un radical alkyle de 1 à 3 atomes de carbone ;
   - ou bien R₄ et R₅ forment ensemble avec la liaison -N-C(=Y)- un cycle de type pyrrolidine, pyrrolidinone, piperidine ou piperidinone ;
   - Y représente deux atomes d'hydrogène ou un hétéroatome comme l'oxygène, le soufre ;
   - Ar représente un cycle 1,4-phényl, 2,5-pyridyl, 5,2-pyridyl ou 2,5-thiophényl ;
   - X représente un atome d'oxygène éventuellement substitué par une chaîne alkyl ou alkylamine ou une liaison simple C-C;
   - A représente un atome d'hydrogène ou la formule suivante : 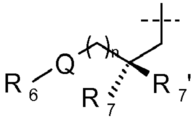 dans laquelle,
      ▪ Q est un atome d'oxygène ou la liaison -NH-;
      ▪ R₆ représente un atome d'hydrogène, un radical alkyle de 1 à 6 atomes de carbone, un radical cycloalkyle de 3 à 6 atomes de carbone, un radical -C(O)CH3 ou-C(O)CH2CH3 ;
      ▪ R₇ et R₇' représentent, indépendamment l'un de l'autre, un atome d'hydrogène ou un groupement hydroxyle, à la condition que R₇ et R₇' ne soient pas simultanément un groupement hydroxyle ;
      ▪ n vaut 0, 1, 2, 3, 4 ou 5 ;
   et les sels des composés de formule (I) lorsque R₃ représente un atome d'hydrogène ainsi que les isomères géométriques desdits composés de formule (I),
b) au moins un agent gélifiant,
c) au moins une silice hydrophobe, et
d) de l'eau
dans laquelle la quantité de principe actif ayant pénétré dans l'épiderme et le derme 16 heures après application topique représente entre 0,1% et 0,8% en poids de la quantité appliquée et préférentiellement entre 0,2% et 0,4% en poids de la quantité appliquée.

De façon encore plus préférée, l'invention porte sur une composition pharmaceutique comprenant :
a) l'acide 3"-*tert*-butyl-4'-(2-hydroxy-ethoxy)-4"-pyrrolidin-1-yl-[1,1';3',1"]-terphenyl-4-carboxylique,
b) au moins un agent gélifiant,
c) au moins une silice hydrophobe, et
d) de l'eau
dans laquelle la quantité de principe actif ayant pénétré dans l'épiderme et le derme 16 heures après application topique représente entre 0,1% et 0,8% en poids de la quantité appliquée et préférentiellement entre 0,2% et 0,4% en poids de la quantité appliquée.

Comparativement à un gel de référence dans lequel le rétinoïde est solubilisé, les compositions selon l'invention libèrent un pourcentage de rétinoïde environ 3 à 4 fois plus faible à dose appliquée équivalente. Cette pénétration du principe actif de la classe des rétinoïdes en plus faible quantité au niveau de l'épiderme et du derme à partir des compostions sous forme de suspension selon l'invention présente l'avantage de minimiser l'effet irritant du à cette classe de composés.

Cet effet moins irritant des compositions selon l'invention se traduit par une meilleure tolérance. L'amélioration de cette tolérance apportée par une composition selon l'invention, c'est-à-dire par une composition pharmaceutique sous forme d'une suspension homogène d'un principe actif de la classe des rétinoïdes dans un gel aqueux comprenant une quantité efficace de silice hydrophobe, est illustrée, à titre d'exemple, sur la Figure 6 (voir exemple 11).

L'irritation observée pour une composition sous forme de suspension de l'acide 3"-*tert*-butyl-4'-(2-hydroxy-ethoxy)-4"-pyrrolidin-1-yl-[1,1';3',1"]-terphenyl-4-carboxylique dans un gel aqueux telle que décrite à l'exemple 1 est comparée avec celle d'une formulation commerciale de Zorac® gel contenant du Tazarotène et avec celle d'un gel de référence contenant de l'acide 3"-*tert*-butyl-4'-(2-hydroxy-ethoxy)-4"-pyrrolidin-1-yl-[1,1';3',1"]-terphenyl-4-carboxylique solubilisé.

Ainsi, dans les conditions du test telles que décrites à l'exemple 11, une composition pharmaceutique sous forme d'une suspension homogène d'un principe actif de la classe des rétinoïdes dans un gel aqueux comprenant une quantité efficace de silice hydrophobe présente une meilleure tolérance comparativement au gel commercial Zorac® ou comparativement à un gel dans lequel le principe actif de la classe des rétinoïdes est solubilisé. L'appréciation de la tolérance est mesurée en utilisant l'index quotidien d'irritation, l'index hebdomadaire d'irritation ou encore l'index cumulé d'irritation.

De préférence, l'invention porte sur une composition pharmaceutique comprenant :
a) un principe actif de la classe des rétinoïdes,
b) au moins un agent gélifiant,
c) au moins une silice hydrophobe, et
d) de l'eau,
composition pour laquelle l'index cumulé d'irritation est inférieur à 1,5 et préférentiellement inférieur à 0,5.

De préférence, l'invention porte sur une composition pharmaceutique comprenant :
a) un principe actif de la classe des rétinoïdes représenté par la formule générale(I) 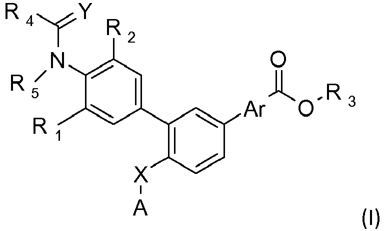 dans laquelle :
   - R₁ est un atome d'hydrogène, un radical alkyle de 1 à 4 atomes de carbone ou un radical-CF₃;
   - R₂ est un atome d'hydrogène, un radical alkyle ou alkoxy de 1 à 4 atomes de carbone ou un atome de chlore ;
   - R₃ est un atome d'hydrogène, un radical alkyle ou alkoxy de 1 à 10 atomes de carbone et de préférence 1 à 6 atomes de carbone, linéaire ou ramifié éventuellement substitué par un groupement méthoxy, ou encore un radical alkyle de 1 à 10 atomes de carbone et de préférence 1 à 6 atomes de carbone, linéaire ou ramifié, contenant une fonction éther ;
   - R₄ est un atome d'hydrogène, un radical alkyle de 1 à 3 atomes de carbone ;
   - R₅ est un atome d'hydrogène, un radical alkyle de 1 à 3 atomes de carbone ;
   - ou bien R₄ et R₅ forment ensemble avec la liaison -N-C(=Y)- un cycle de type pyrrolidine, pyrrolidinone, piperidine ou piperidinone ;
   - Y représente deux atomes d'hydrogène ou un hétéroatome comme l'oxygène, le soufre ;
   - Ar représente un cycle 1,4-phényl, 2,5-pyridyl, 5,2-pyridyl ou 2,5-thiophényl ;
   - X représente un atome d'oxygène éventuellement substitué par une chaîne alkyl ou alkylamine ou une liaison simple C-C;
   - A représente un atome d'hydrogène ou la formule suivante : 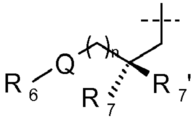 dans laquelle,
      ▪ Q est un atome d'oxygène ou la liaison -NH-;
      ▪ R₆ représente un atome d'hydrogène, un radical alkyle de 1 à 6 atomes de carbone, un radical cycloalkyle de 3 à 6 atomes de carbone, un radical -C(O)CH3 ou-C(O)CH2CH3 ;
      ▪ R₇ et R₇' représentent, indépendamment l'un de l'autre, un atome d'hydrogène ou un groupement hydroxyle, à la condition que R₇ et R₇' ne soient pas simultanément un groupement hydroxyle ;
      ▪ n vaut 0, 1, 2 , 3, 4 ou 5 ;
   et les sels des composés de formule (I) lorsque R₃ représente un atome d'hydrogène ainsi que les isomères géométriques desdits composés de formule (I),
b) au moins un agent gélifiant,
c) au moins une silice hydrophobe, et
d) de l'eau,
composition pour laquelle l'index cumulé d'irritation est inférieur à 1,5 et préférentiellement inférieur à 0,5.

De façon encore plus préférée, l'invention porte sur une composition pharmaceutique comprenant :
a) l'acide 3"-*tert*-butyl-4'-(2-hydroxy-ethoxy)-4"-pyrrolidin-1-yl-[1,1';3',1"]-terphenyl-4-carboxylique,
b) au moins un agent gélifiant,
c) au moins une silice hydrophobe, et
d) de l'eau,
composition pour laquelle l'index cumulé d'irritation est inférieur à 1,5 et préférentiellement inférieur à 0,5.

Les compositions selon l'invention présentent d'excellentes propriétés de stabilité chimique et de stabilité physique.

Les exemples suivants sont destinés à illustrer l'invention sans aucunement en limiter la portée.

A moins qu'il n'en soit précisé autrement, les pourcentages indiqués dans les exemples suivants sont des pourcentages en poids par rapport au poids total de la composition concernée.

### EXEMPLES :

### 1- Essais de dispersion de l'acide 3"-tert-butyl-4'-(2-hydroxy-ethoxy)-4"-pyrrolidin-1-yl-[1,1;3',1"]-terphenyl-4-carboxylique à l'aide de "non-solvant" :

Le but de ces essais est d'obtenir des formulations dans lesquelles le principe actif de la classe des rétinoïdes est dispersé de façon homogène dans un "non-solvant" (i.e. pas d'agglomérats ou d'amas, taille régulière de la dispersion) et qui présentent une bonne stabilité physique (i.e. le principe actif reste sous sa forme dispersée et ne se solubilise pas dans le temps).

Pour ces essais, l'acide 3"-tert-butyl-4'-(2-hydroxy-ethoxy)-4"-pyrrolidin-1-yl-[1,1';3',1"]-terphenyl-4-carboxylique est utilisé à titre d'exemple de principe actif de la classe des rétinoïdes.

Ce rétinoïde est pré-mélangé sous forme solide avec chacun des non solvants listés dans le tableau 6 ci-dessous, et ce pré-mélange est additionné à un gel aqueux de faible viscosité dans le but de permettre une mise en oeuvre aisée du principe actif et d'obtenir un bon maintien en suspension de celui-ci (pas de sédimentation, ni d'agglomérat). Le gel aqueux utilisé est à base de Simulgel 600. Toutefois, tous les essais réalisés en utilisant un « non-solvant » du principe actif de la classe des rétinoïdes n'ont pas permis d'obtenir une suspension homogène comme résumé dans le tableau 6 ci-dessous.

**Tableau 6 : Essais avec des « non-solvants » de l'acide 3"-tert-butyl-4'-(2-hydroxy-ethoxy)-4"-pyrrolidin-1-yl-[1,1';3',1"]-terphenyl-4-carboxylique.**

| **NON-SOLVANTS** | **Type de non-solvant** | **RESULTATS** |
|---|---|---|
| EAU PURIFIEE | Hydrophiles | Résultat non satisfaisant (obtention de 2 phases distinctes) |
| GLYCERINE | | |
| ISOPROPYL MYRISTATE | Lipophiles | La dispersion est très hétérogène. Les particules d'acide 3"-tert-butyl-4'-(2-hydroxy-ethoxy)-4"-pyrrolidin-1-yl-[1,1';3',1"]-terphenyl-4-carboxylique sont rassemblées dans des grosses gouttelettes d'huile qu'il n'est pas possible de réduire. |
| COSBIOL | | |
| ST-CYCLOMETHICONE 5-NF | | |
| MARCOL 152 | | |
| PRIMOL 352 | | |

### 1-a) Formule avec la Glycérine

| **DENOMINATION COMMERCIALE** | **DENOMINATION INCI** | **%** |
|---|---|---|
| Acide 3"-tert-butyl-4'-(2-hydroxy-ethoxy)-4"-pyrrolidin-1-yl-[1,1';3',1"]-terphenyl-4-carboxylique | 3"-tert-butyl-4'-(2-hydroxy-ethoxy)-4"-pyrrolidin-1-yl-[1,1';3',1"]-terphenyl-4-carboxylic acid | 0,10 |
| GLYCERINE | Glycerin | 10,00 |
| ST-CYCLOMETHICONE 5-NF | Cyclopentasiloxane | 2,00 |
| SIMULGEL 600 PHA | Acrylamide, AMPS Copolymer Dispersion 40%/ Isohexadecane / Polysorbate 80 | 2,10 |
| EAU PURIFIEE | Purified water | QSP 100.00 |

| | | |
|---|---|---|
| **CARACTERISATION À T0** | **ASPECT MACROSCOPIQUE** | **Crème blanche, lisse, brillante** |
| | **ASPECT MICROSCOPIQUE** (voir Figure 7) | **Dispersion hétérogène** |

### 1-b) Formule avec Marcol 152

| **DENOMINATION COMMERCIALE** | **DENOMINATION INCI** | **%** |
|---|---|---|
| Acide 3"-tert-butyl-4'-(2-hydroxy-ethoxy)-4"-pyrrolidin-1-yl-[1,1';3',1"]-terphenyl-4-carboxylique (micronisé) | 3"-tert-butyl-4'-(2-hydroxy-ethoxy)-4"-pyrrolidin-1-yl-[1,1';3',1"]-terphenyl-4-carboxylic acid (micronised) | 0,01 |
| MARCOL 152 | Mineral oil | 0,20 |
| ACIDE BENZOIQUE | Benzoic acid | 0,10 |
| SORBATE DE POTASSIUM | Potassium Sorbate | 0,10 |
| SIMULGEL 600 PHA | Acrylamide, AMPS Copolymer Dispersion 40%/ Isohexadecane / Polysorbate 80 | 3,00 |
| EAU PURIFIEE | Purified water | QSP 100.00 |

| | | |
|---|---|---|
| **CARACTERISATION À T0** | **ASPECT MACROSCOPIQUE** | **Crème blanche, lisse, brillante** |
| | **ASPECT MICROSCOPIQUE** (voir Figure 1) | **Dispersion hétérogène** |

Ces essais de dispersion directe de l'acide 3"-tert-butyl-4'-(2-hydroxy-ethoxy)-4"-pyrrolidin-1-yl-[1,1';3',1"]-terphenyl-4-carboxylique dans des non-solvants ne permettent pas d'obtenir une bonne dispersion des particules solides du principe actif de la classe des rétinoïdes dans un gel aqueux.

### 2 - Essais de dispersion de l'acide 3"-tert-butyl-4'-(2-hydroxy-ethoxy)-4"-pyrrolidin-1-yl-[1,1;3',1"]-terphenyl-4-carboxylique avec des agents mouillants :

Pour ces essais, l'acide 3"-tert-butyl-4'-(2-hydroxy-ethoxy)-4"-pyrrolidin-1-yl-[1,1';3',1"]-terphenyl-4-carboxylique, utilisé à titre d'exemple de principe actif de la classe des rétinoïdes, est pré-mélangé sous forme solide avec des agents mouillant, seuls ou en association avec des non-solvants :

| **Agents mouillant** | **Résultats** |
|---|---|
| Glycerine/Propylene Glycol | Mauvaise dispersion de l'actif et solubilisation partielle. |
| Polysorbate 60 | Solubilisation de l'acide 3"-tert-butyl-4'-(2-hydroxy-ethoxy)-4"-pyrrolidin-1-yl-[1,1';3',1"]-terphenyl-4-carboxylique |
| Pluronic/Glycerine/Cyclomethicone | Très mauvaise dispersion de l'actif. Présence de nombreux agrégats |

### 2-a) Formule avec Glycérine/ Propylène Glycol

| **DENOMINATION COMMERCIALE** | **DENOMINATION INCI** | **%** |
|---|---|---|
| Acide 3"-tert-butyl-4'-(2-hydroxy-ethoxy)-4"-pyrrolidin-1-yl-[1,1';3',1"]-terphenyl-4-carboxylique | 3"-tert-butyl-4'-(2-hydroxy-ethoxy)-4"-pyrrolidin-1-yl-[1,1';3',1"]-terphenyl-4-carboxylic acid | 0,10 |
| GLYCERINE | Glycerin | 4,00 |
| PROPYLENE GLYCOL | Propylene Glycol | 2,00 |
| SIMULGEL 600 PHA | Acrylamide, AMPS Copolymer Dispersion 40%/ Isohexadecane / Polysorbate 80 | 4,00 |
| EAU PURIFIEE | Purified water | QSP 100.00 |

| | | |
|---|---|---|
| **CARACTERISATION À T0** | **ASPECT MACROSCOPIQUE** | **Crème blanche, lisse, brillante** |
| | **ASPECT MICROSCOPIQUE** (voir Figure 8) | **Taille des particules hétérogène 15µm< ∅ <100µm** |

### 2-b) Formule avec Polysorbate 60

| **DENOMINATION COMMERCIALE** | **DENOMINATION INCI** | % |
|---|---|---|
| Acide 3"-tert-butyl-4'-(2-hydroxy-ethoxy)-4"-pyrrolidin-1-yl-[1,1';3',1"]-terphenyl-4-carboxylique | 3"-tert-butyl-4'-(2-hydroxy-ethoxy)-4"-pyrrolidin-1-yl-[1,1';3',1"]-terphenyl-4-carboxylic acid | 0,01 |
| TWEEN 60 | Polysorbate 60 | 0,09 |
| ACIDE BENZOIQUE | Benzoic acid | 0,10 |
| SORBATE DE POTASSIUM | Potassium Sorbate | 0,10 |
| SIMULGEL 600 PHA | Acrylamide, AMPS Copolymer Dispersion 40%/ Isohexadecane / Polysorbate 80 | 3,00 |
| EAU PURIFIEE | Purified water | QSP 100.00 |

| | | |
|---|---|---|
| **CARACTERISATION À T0** | **ASPECT MACROSCOPIQUE** | **Crème Blanche, lisse, brillante** |
| | **ASPECT MICROSCOPIQUE** (voir Figure 9) | **Très peu de cristaux de principe actif (principe actif solubilisé) Présence de quelques agglomérats** |

### 2-c) Formule avec Pluronic/Glycérine/Cyclomethicone

| **DENOMINATION COMMERCIALE** | **DENOMINATION INCI** | **%** |
|---|---|---|
| Acide 3"-tert-butyl-4'-(2-hydroxy-ethoxy)-4"-pyrrolidin-1-yl-[1,1';3',1"]-terphenyl-4-carboxylique | 3"-tert-butyl-4'-(2-hydroxy-ethoxy)-4"-pyrrolidin-1-yl-[1,1';3',1"]-terphenyl-4-carboxylic acid | 0,01 |
| PLURONIC L44 | Poloxamer 124 | 0,10 |
| GLYCERINE | Glycerin | 1,00 |
| ST-CYCLOMETHICONE5-NF | Cyclopentasiloxane | 2,00 |
| RONACARE ALLANTOINE | Allantoin | 0,20 |
| SIMULGEL 600 PHA | Acrylamide, AMPS Copolymer Dispersion 40%/ Isohexadecane / Polysorbate 80 | 2,00 |
| EAU PURIFIEE | Purified water | QSP 100.00 |

| | | |
|---|---|---|
| **CARACTERISATION À T0** | **ASPECT MACROSCOPIQUE** | **Crème blanche, lisse, brillante** |
| | **ASPECT MICROSCOPIQUE** (voir Figure 3) | **Présence de particules et d'agglomérats. 10µm < ∅ < 250µm Dispersion très hétérogène** |

Ces essais montrent que les agents mouillants évalués :
- soit ne permettent pas d'obtenir une bonne dispersion des particules,
- soit solubilisent partiellement l'acide 3"-tert-butyl-4'-(2-hydroxy-ethoxy)-4"-pyrrolidin-1-yl-[1,1';3',1"]-terphenyl-4-carboxylique.

### 3 - Exemples de formulations:

Dans les exemples suivants, les formules réalisées sont caractérisées à T0 à l'aide du matériel et des méthodes décrits ci-dessous. La stabilité physique et la stabilité chimique des formulations sont réalisées après conservation à température ambiante (TA) et à +40°C après T0+1Mois et/ou T0+2Mois et/ou T0+3Mois et/ou T0+6Mois. L'acide 3"-tert-butyl-4'-(2-hydroxy-ethoxy)-4"-pyrrolidin-1-yl-[1,1';3',1"]-terphenyl-4-carboxylique est utilisé à titre d'exemple de principe actif de la classe des rétinoïdes.

### - Dosage chimique de l'acide 3"-tert-butyl-4'-(2-hydroxy-ethoxy)-4"-pyrrolidin-1-yl-[1,1';3',1"]-terphenyl-4-carboxylique:

- Matériel: HPLC
- Expression des résultats: le titre de l'actif est exprimé en % relatif par rapport au % initial effectué à T0. Les limites fixées pour une bonne stabilité chimique sont 95%-105%.

### -Observation macroscopique:

- L'observation macroscopique permet de garantir l'intégrité physique des produits à T0 et après stabilité.

### -Observation microscopique:

- L'observation microscopique permet d'évaluer la bonne dispersion (homogénéité) de l'acide 3"-tert-butyl-4'-(2-hydroxy-ethoxy)-4"-pyrrolidin-1-yl-[1,1';3',1"]-terphenyl-4-carboxylique dès T0 ainsi que la bonne stabilité physique au cours du temps (maintien de l'homogénéité de la dispersion)
- Matériel: Microscope AXIO ZEISS

### -pH:

- Matériel: pH mètre METTLER TOLEDO Seven Multi
- Méthode : Mesures effectuées à température ambiante après stabilisation 24h dans une enceinte à 25°C de tous les échantillons.

### -Viscosité:

- La mesure de la viscosité permet d'évaluer la consistance des formules réalisées.
- Matériel: Brookfield RV DVII + Pro
- Méthode: Mesures effectuées à température ambiante après stabilisation 24h dans une enceinte à 25°C de tous les échantillons. La valeur est lue après 1 minute. Le choix du mobile et de la vitesse seront décrits dans chaque exemple de composition. Les valeurs obtenues sont exprimées en centipoises (Cps)

### -Centrifugation:

- La centrifugation permet d'évaluer la résistance des formules à une contrainte mécanique.
- Matériel: Galaxy 14D VWR
- Méthode: 30 minutes à 5000 rpm

### 3-a) Exemple 1.

| **DENOMINATION COMMERCIALE** | **DENOMINATION INCI** | **%** |
|---|---|---|
| Acide 3"-tert-butyl-4'-(2-hydroxy-ethoxy)-4"-pyrrolidin-1-yl-[1,1';3',1"]-terphenyl-4-carboxylique (micronisé) | 3"-tert-butyl-4'-(2-hydroxy-ethoxy)-4"-pyrrolidin-1-yl-[1,1';3',1"]-terphenyl-4-carboxylic acid (micronized) | 0,008 |
| Aerosil® R972 | Silica Dimethyl Silylate | 0,002 |
| ACIDE BENZOIQUE | Benzoic acid | 0,060 |
| SORBATE DE POTASSIUM | Potassium Sorbate | 0,060 |
| SIMULGEL 600 PHA | Acrylamide, AMPS Copolymer Dispersion 40%/ Isohexadecane / Polysorbate 80 | 3,000 |
| EAU PURIFIEE | Purified water | QSP 100.000 |

| | | |
|---|---|---|
| **CARACTERISATION À T0** | **ASPECT MACROSCOPIQUE** | **Crème Blanche, lisse, brillante** |
| | **ASPECT MICROSCOPIQUE** (voir Figure 4) | **Présence de particules. 2µm < Ø < 10µm** |

### 3-b) Exemple 2.

| **DENOMINATION COMMERCIALE** | **DENOMINATION INCI** | % |
|---|---|---|
| Acide 3"-tert-butyl-4'-(2-hydroxy-ethoxy)-4"-pyrrolidin-1-yl-[1,1';3',1"]-terphenyl-4-carboxylique | 3"-tert-butyl-4'-(2-hydroxy-ethoxy)-4"-pyrrolidin-1-yl-[1,1';3',1"]-terphenyl-4-carboxylic acid | 0,0100 |
| Aerosil® R972 | Silica Dimethyl Silylate | 0,0025 |
| ACIDE BENZOIQUE | Benzoic acid | 0,100 |
| SORBATE DE POTASSIUM | Potassium Sorbate | 0,100 |
| SIMULGEL 600 PHA | Acrylamide, AMPS Copolymer Dispersion 40%/ Isohexadecane / Polysorbate 80 | 3,000 |
| EAU PURIFIEE | Purified water | QSP 100.000 |

| | | |
|---|---|---|
| **CARACTERISATION À T0** | **ASPECT MACROSCOPIQUE** | **Crème Blanche, lisse, brillante** |
| | **ASPECT MICROSCOPIQUE** | **Présence de particules. 2µm < Ø < 10µm** |

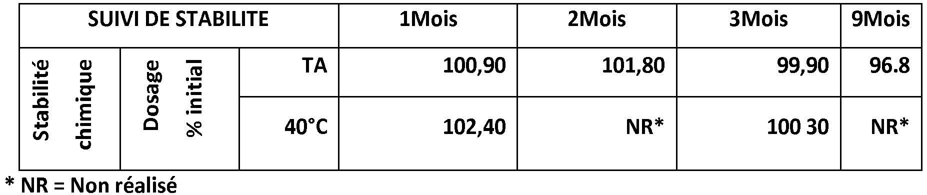

Cet exemple montre que la composition décrite est stable chimiquement au moins 3 mois à température ambiante et 40°C et 9 mois à température ambiante.

### 3-c) Exemple 3.

| **DENOMINATION COMMERCIALE** | **DENOMINATION INCI** | **%** |
|---|---|---|
| Acide 3"-tert-butyl-4'-(2-hydroxy-ethoxy)-4"-pyrrolidin-1-yl-[1,1';3',1"]-terphenyl-4-carboxylique | 3"-tert-butyl-4'-(2-hydroxy-ethoxy)-4"-pyrrolidin-1-yl-[1,1';3',1"]-terphenyl-4-carboxylic acid | 0,0100 |
| Aerosil® R972 | Silica Dimethyl Silylate | 0,0025 |
| MARCOL 152 | Mineral oil | 0,200 |
| ACIDE BENZOIQUE | Benzoic acid | 0,080 |
| SORBATE DE POTASSIUM | Potassium Sorbate | 0,080 |
| SIMULGEL 600 PHA | Acrylamide, AMPS Copolymer Dispersion 40%/ Isohexadecane / Polysorbate 80 | 3,000 |
| EAU PURIFIEE | Purified water | QSP 100.000 |

| | | |
|---|---|---|
| **CARACTERISATION À T0** | **ASPECT MACROSCOPIQUE** | **Crème Blanche, lisse, brillante** |
| | **ASPECT MICROSCOPIQUE** (voir Figure 2) | **Présence de particules. 2µm < Ø < 10µm** |

### 3-d) Exemple 4.

| **DENOMINATION COMMERCIALE** | **DENOMINATION INCI** | % |
|---|---|---|
| Acide 3"-tert-butyl-4'-(2-hydroxy-ethoxy)-4"-pyrrolidin-1-yl-[1,1';3',1"]-terphenyl-4-carboxylique | 3"-tert-butyl-4'-(2-hydroxy-ethoxy)-4"-pyrrolidin-1-yl-[1,1';3',1"]-terphenyl-4-carboxylic acid | 0,0500 |
| Aerosil® R972 | Silica Dimethyl Silylate | 0,0125 |
| MARCOL 152 | Mineral oil | 0,200 |
| ACIDE BENZOIQUE | Benzoic acid | 0,080 |
| SORBATE DE POTASSIUM | Potassium Sorbate | 0,080 |
| SIMULGEL 600 PHA | Acrylamide, AMPS Copolymer Dispersion 40%/ Isohexadecane / | 3,000 |
| | Polysorbate 80 | |
| EAU PURIFIEE | Purified water | QSP 100.000 |

| | | |
|---|---|---|
| **CARACTERISATION À T0** | **ASPECT MACROSCOPIQUE** | **Crème Blanche, lisse, brillante** |
| | **ASPECT MICROSCOPIQUE** (voir Figure 10) | **Présence de particules. 2µm < Ø < 10µm** |

### 3-e) Exemple 5.

| **DENOMINATION COMMERCIALE** | **DENOMINATION INCI** | % |
|---|---|---|
| Acide 3"-tert-butyl-4'-(2-hydroxy-ethoxy)-4"-pyrrolidin-1-yl-[1,1';3',1"]-terphenyl-4-carboxylique | 3"-tert-butyl-4'-(2-hydroxy-ethoxy)-4"-pyrrolidin-1-yl-[1,1';3',1"]-terphenyl-4-carboxylic acid | 0,0500 |
| Aerosil® R972 | Silica Dimethyl Silylate | 0,0125 |
| COSBIOL | Squalane | 0.500 |
| CHLORURE DE BENZALKONIUM | benzalkonium chloride | 0,100 |
| PEMULEN TR1 | Acrylates/C10-30 Alkyl Acrylate Crosspolymer | 0.500 |
| EAU PURIFIEE | Purified water | QSP 100.000 |

### 3-f) Exemple 6.

| **DENOMINATION COMMERCIALE** | **DENOMINATION INCI** | % |
|---|---|---|
| Acide 3"-tert-butyl-4'-(2-hydroxy-ethoxy)-4"-pyrrolidin-1-yl-[1,1';3',1"]-terphenyl-4-carboxylique | 3"-tert-butyl-4'-(2-hydroxy-ethoxy)-4"-pyrrolidin-1-yl-[1,1';3',1"]-terphenyl-4-carboxylic acid | 0,0500 |
| Aerosil® R972 | Silica Dimethyl Silylate | 0,0125 |
| COSBIOL | Squalane | 0.500 |
| NIPASOL | Propylparaben | 0.050 |
| NIPAGIN M | Methylparaben | 0.100 |
| PEMULEN TR2 | Acrylates/C10-30 Alkyl Acrylate Crosspolymer | 0.300 |
| EAU PURIFIEE | Purified water | QSP 100.000 |

### 3-g) Exemple 7.

| **DENOMINATION COMMERCIALE** | **DENOMINATION INCI** | % |
|---|---|---|
| Acide 3"-tert-butyl-4'-(2-hydroxy-ethoxy)-4"-pyrrolidin-1-yl-[1,1';3',1"]-terphenyl-4-carboxylique | 3"-tert-butyl-4'-(2-hydroxy-ethoxy)-4"-pyrrolidin-1-yl-[1,1';3',1"]-terphenyl-4-carboxylic acid | 0,0500 |
| Aerosil® R972 | Silica Dimethyl Silylate | 0,0125 |
| NIPASOL | Propylparaben | 0.050 |
| NIPAGIN M | Methylparaben | 0.100 |
| PEMULEN TR2 | Acrylates/C10-30 Alkyl Acrylate Crosspolymer | 0.500 |
| EAU PURIFIEE | Purified water | QSP 100.000 |

### 3-h) Exemple 8.

| **DENOMINATION COMMERCIALE** | **DENOMINATION INCI** | % |
|---|---|---|
| Acide 3"-tert-butyl-4'-(2-hydroxy-ethoxy)-4"-pyrrolidin-1-yl-[1,1';3',1"]-terphenyl-4-carboxylique | 3"-tert-butyl-4'-(2-hydroxy-ethoxy)-4"-pyrrolidin-1-yl-[1,1';3',1"]-terphenyl-4-carboxylic acid | 0,100 |
| Aerosil® R972 | Silica Dimethyl Silylate | 0,025 |
| ACIDE BENZOIQUE | Benzoic acid | 0,100 |
| SORBATE DE POTASSIUM | Potassium Sorbate | 0,100 |
| SIMULGEL 600 PHA | Acrylamide, AMPS Copolymer Dispersion 40%/ Isohexadecane / Polysorbate 80 | 3,000 |
| EAU PURIFIEE | Purified water | QSP 100.000 |

| | | |
|---|---|---|
| **CARACTERISATION À T0** | **ASPECT MACROSCOPIQUE** | **Crème Blanche, lisse, brillante** |
| | **ASPECT MICROSCOPIQUE** | **Présence de particules. 2µm < ∅ < 10µm** |

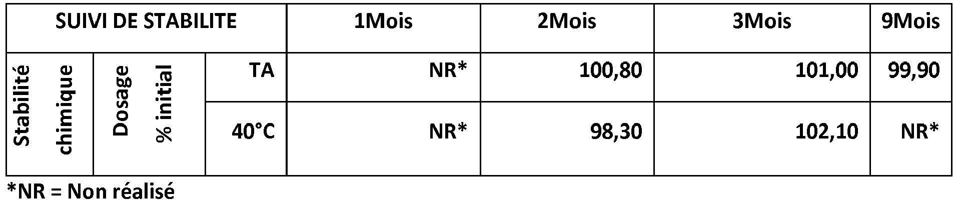

Cet exemple montre que la composition décrite est stable chimiquement au moins 3 mois à température ambiante et 40°C et 9 mois à température ambiante.

### 3-i) Exemple 9.

| **DENOMINATION COMMERCIALE** | **DENOMINATION INCI** | % |
|---|---|---|
| Acide 3"-tert-butyl-4'-(2-hydroxy-ethoxy)-4"-pyrrolidin-1-yl-[1,1';3',1"]-terphenyl-4-carboxylique | 3"-tert-butyl-4'-(2-hydroxy-ethoxy)-4"-pyrrolidin-1-yl-[1,1';3',1"]-terphenyl-4-carboxylic acid | 0,0500 |
| Aerosil® R972 | Silica Dimethyl Silylate | 0,0125 |
| ACIDE BENZOIQUE | Benzoic acid | 0,100 |
| SORBATE DE POTASSIUM | Potassium Sorbate | 0,100 |
| SIMULGEL 600 PHA | Acrylamide, AMPS Copolymer Dispersion 40%/ Isohexadecane / Polysorbate 80 | 3,000 |
| EAU PURIFIEE | Purified water | QSP 100.000 |

| | | |
|---|---|---|
| **CARACTERISATION À T0** | **ASPECT MACROSCOPIQUE** | **Crème Blanche, lisse, brillante** |
| | **ASPECT MICROSCOPIQUE** | **Présence de particules. 2µm < ∅ < 10µm** |

### 4- Exemple 10 : Etudes in vitro de pénétration cutanée sur peau humaine:

Ces études permettent de caractériser les formulations de l'invention en déterminant *in vitro* la pénétration du principe actif de la classe des rétinoïdes dans les différents compartiments de la peau. Elles permettent également d'établir une classification des formulations de l'invention selon qu'elles pourront promouvoir ou limiter la pénétration du principe actif, ou cibler un compartiment particulier de la peau. La capacité d'une formulation à faire pénétrer un composé constitue une des caractéristiques clés en vue d'une application topique, pour laquelle la peau constitue une barrière majeure.

Les études de pénétration cutanée réalisées sur les formulations de l'invention contenant un principe actif de la classe des rétinoïdes ont été réalisées *in vitro* sur de la peau humaine abdominale entière après excision, cette peau étant montée sur une cellule de Franz.

Après application topique d'une quantité donnée de la formulation à la surface de la peau, la pénétration du principe actif est mesurée 16h après application.

La quantité de principe actif de la classe des rétinoïdes est mesurée dans les différents compartiments de la peau : stratum corneum, épiderme, derme et également dans le liquide récepteur.

A titre d'exemple, la pénétration du principe actif des formulations des exemples 2 et 9 a été évaluée, et le détail de ces études de pénétration est donné dans le tableau ci-dessous.

Les formulations évaluées sont les gels dispersés de l'exemple 2, contenant 100µg/g d'acide 3"-tert-butyl-4'-(2-hydroxy-ethoxy)-4"-pyrrolidin-1-yl-[1,1';3',1"]-terphenyl-4-carboxylique, et de l'exemple 9, contenant 500µg/g de ce même principe actif.

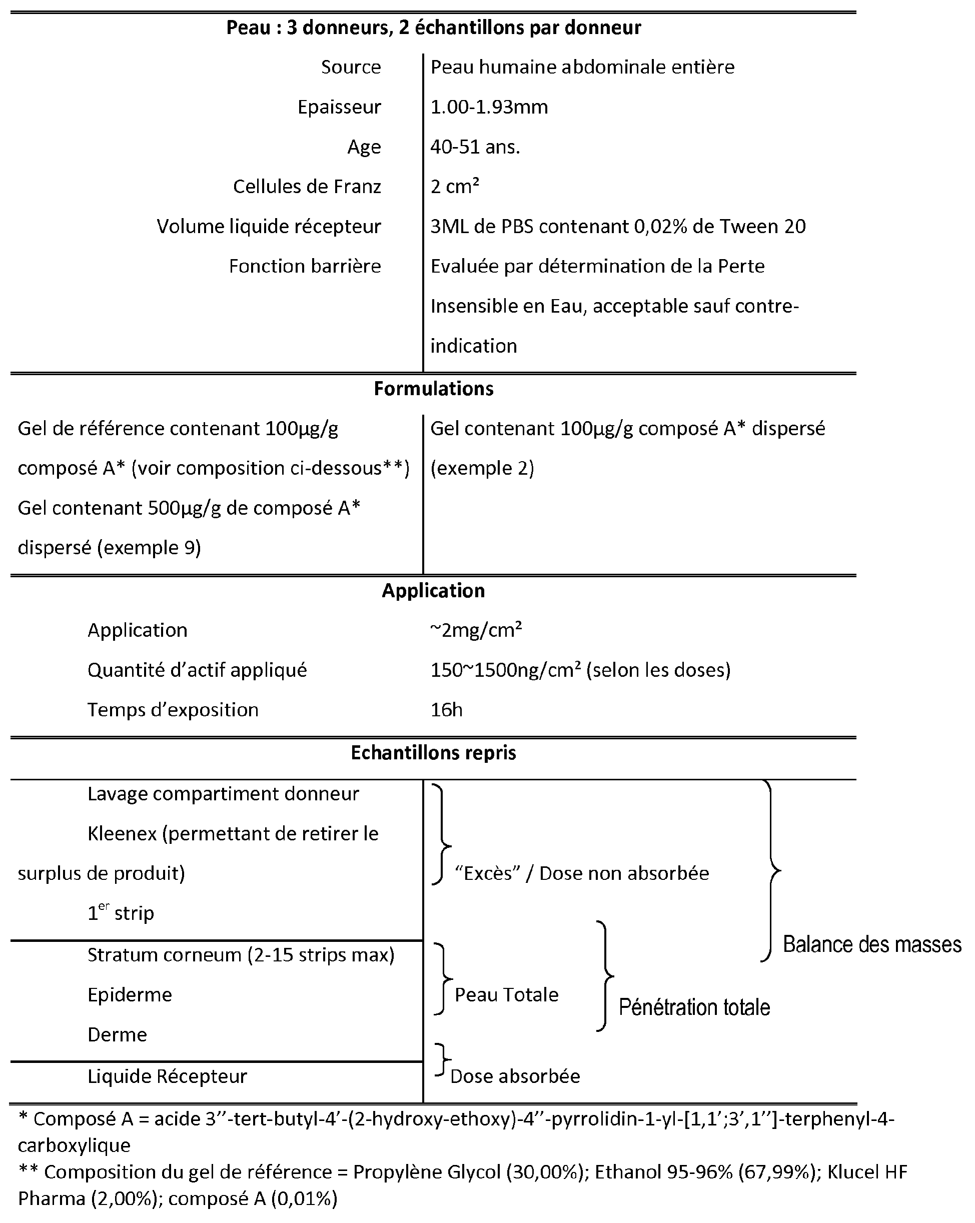

La bioanalyse a été réalisée par spectrométrie de masse en tandem par ionisation electrospray positive, et en utilisant un appareil Xevo (Waters).

Les conditions techniques sont données dans le tableau ci-dessous.

| | | | | | | |
|---|---|---|---|---|---|---|
| Colonne LC | Hypersil gold 50*2.1mm (UPLC) | | | | | |
| Phase Mobile | Phase A : ACN + 0.1% Formic Acid | | | | | |
| | Phase B : H₂O + 0.1% Formic acid | | | | | |
| Lavage aiguille | ACN | | | | | |
| Lavage septum | ACN/H₂O (50:50) | | | | | |
| Gradient | Temps (min) | débit | %A | %B | Courbe | |
| | 1. Initial | 0.700 | 15.0 | 85.0 | 0 | |
| | 2.2.5 | 0.700 | 90.0 | 10.0 | 6 | |
| | 3.3.20 | 0.700 | 90.0 | 10.0 | 6 | |
| | 4.3.25 | 0.700 | 15.0 | 85.0 | 6 | |
| Detection MS/MS | ESI+ MRM (Electrospray Positif) | | | | | |
| | | | | | | |
| | Canal Réaction | Dwell (secs) | Voltage (cône) | Col. Energy | Tr (min) | Composé |
| | 1: 460.26 > 318.20 | 0.100 | 50.0 | 40.0 | 1.58 | Composé A |
| | 1: 464.06 > 372.10 | 0.100 | 55.0 | 40.0 | 1.58 | Standard Interne Deutéré |
| Volume Injection | 5µL | | | | | |
| Temps de run | 4 minutes | | | | | |

La limite de quantification pour le composé A est de 0.5ng/mL. Les conditions de LC/MS/MS mises au point ont permis de détecter jusqu'à 0,1% de la dose appliquée dans chacun des compartiments (dose non absorbée, stratum, épiderme, derme et liquide récepteur). La justesse, la précision et la répétabilité de la méthode ont été vérifiées dans chacune des matrices, et ont permis de valider la méthode de bio analyse.

Dans ce type d'étude « point unique », les paramètres retenus sont :
a. Le profil de distribution dans les différents compartiments (données qualitatives)
b. La pénétration dans le compartiment épiderme + derme (données numériques)

### a. Profil de distribution dans les différents compartiments : voir Figure 5

La distribution entre les différents compartiments est du même type pour les 3 formules évaluées : accumulation dans le stratum corneum, taux de pénétration plus faible dans l'épiderme, et pénétration très faible dans le derme. Le composé A n'est pas détecté dans le liquide récepteur. La pénétration du composé A à partir d'un gel dispersé est plus faible à dose équivalente que le gel de référence. Lorsqu'on augmente la concentration du composé A dispersé à 500µg/g dans le gel, les niveaux de pénétration tendent à être similaires à ceux obtenus après application du gel de référence. La dispersion du composé A dans un gel permet donc de minimiser le taux de pénétration.

### b. Valeurs de pénétration dans le compartiment épiderme + derme :

Les valeurs de pénétration pour le gel contenant 100µg/g (0.01%) de composé A dispersé est environ de 0,60ng/cm²,

La pénétration du composé A après application du gel dispersé contenant 500µg/g (0,05%) est environ de 3.45ng/cm²,

Cette étude montre que pour une composition pharmaceutique sous forme d'une suspension homogène d'un principe actif de la classe des rétinoïdes dans un gel aqueux comprenant une quantité efficace de silice hydrophobe, la quantité de principe actif ayant pénétré dans l'épiderme et le derme 16 heures après application topique représente entre 0.1% et 0.8% de la quantité appliquée et préférentiellement entre 0,2% et 0,4% de la quantité appliquée.

### 5- Exemple 11: Etude de tolérance cutanée chez le mini porc

### Méthodologie :

4 males et 4 femelles mini porcs Göttingen® âgés de 4 mois environ sont inclus dans chaque étude. Chaque animal est traité quotidiennement sur les flancs (7 jour/ 7 pendant 4 semaines consécutives) par application d'environ 17.8 mg / cm² de formulation sur une zone délimitée. Après application, Les zones traitées sont protégées jusqu'à évaluation des réactions cutanées environ 6 heures après traitement, selon l'échelle de Draize décrite dans le texte réglementaire "OCDE Guideline No. 404. Acute dermal irritation/corrosion".

| formation **d'érythème** et escarres | |
|---|---|
| Pas d'érythème | 0 |
| Erythème très léger (à peine perceptible) | 1 |
| Erythème bien défini | 2 |
| Erythème modéré à grave | 3 |
| Erythème grave (rouge violacé) à formation d'escarre empêchant la cotation de l'érythème | 4 |

| **Formation d'OEdème** | |
|---|---|
| Pas d'OEdème | 0 |
| OEdème très léger (à peine perceptible) | 1 |
| OEdème léger (pourtour de la zone oedémateuse bien délimité par une enflure nette) | 2 |
| OEdème modéré (enflure d'environ 1 mm) | 3 |
| OEdème grave (enflure de plus de 1 mm s'étendant au-delà de l'aire exposée) | 4 |

Pour chaque formulation, un index d'irritation quotidien (Mean Daily Index ou DI), un index d'irritation hebdomadaire (Mean Weekly Index ou WI) et un index d'irritation cumulé (Mean Cumulative Irritancy Index ou MCII) sont calculés.

Les formulations sont classées selon leur score d'irritation de la façon suivante :

| **Index hebdomadaire (WI) ou Index cumulé (MCI)** | |
|---|---|
| 0 | Non-irritant |
| 0.5≥WI ou MCI>0 | Pratiquement pas irritant |
| 2 ≥WI ou MCI>0.5 | Peu irritant |
| 5 ≥WI ou MCI> 2 | Irritant |
| 8 ≥WI ou MCI> 5 | Trés irritant |

### Résultats :

### 1. Tolérance de la formule de l'exemple 1 : voir Figure 6

| **Dénomination Formulation** | **Index hebdomadaire (WI)** | | | | **Index cumulé (MCI)** |
|---|---|---|---|---|---|
| | W1 | W2 | W3 | W4 | |
| Zorac® gel 0.05% (Tazarotene 0,05%) | 0.30 | 1.52 | 2.95 | 3.09 | 1.96 |
| Gel de référence contenant 100µg/g de composé A* (voir composition ci-dessous**) | 0.05 | 0.77 | 2.14 | 2.29 | 1.31 |
| Exemple 1 contenant 100µg/g de composé A* | 0.00 | 0.09 | 0.45 | 0.63 | 0.29 |

| | | | | | |
|---|---|---|---|---|---|
| *Composé A = Acide 3"-tert-butyl-4'-(2-hydroxyethoxy)-4"-pyrrolidin-1-yl[1,1';3',1"] terphenyl-4-carboxylique. ** Composition du gel de référence = Propylène Glycol (30,00%); Ethanol 95-96% (67,99%); Klucel HF Pharma (2,00%); composé A (0,01%) | | | | | |

### Conclusion :

L'exemple 1 est classé «pratiquement pas irritant» et montre une meilleure tolérance que le gel de référence à la même concentration et que Zorac® gel 0.05%.

## Revendications

1. Composition pharmaceutique comprenant
a) un principe actif de la classe des rétinoïdes,
b) au moins un agent gélifiant,
c) au moins une silice hydrophobe, et
d) de l'eau.

2. Composition selon la revendication 1 **caractérisée en ce que** le principe actif de la classe des rétinoïdes se trouve sous forme dispersée.

3. Composition selon la revendication 1 ou la revendication 2 **caractérisée en ce que** le rétinoïde est choisi parmi un des composés représentés par la formule générale (I) suivante : 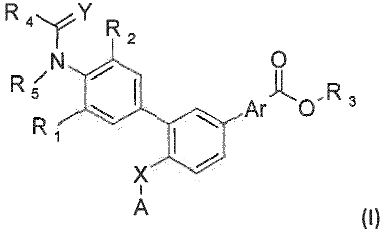 dans laquelle :
- R₁ est un atome d'hydrogène, un radical alkyle de 1 à 4 atomes de carbone ou un radical -CF₃ ;
- R₂ est un atome d'hydrogène, un radical alkyle ou alkoxy de 1 à 4 atomes de carbone ou un atome de chlore ;
- R₃ est un atome d'hydrogène, un radical alkyle ou alkoxy de 1 à 10 atomes de carbone et de préférence 1 à 6 atomes de carbone, linéaire ou ramifié éventuellement substitué par un groupement méthoxy, ou encore un radical alkyle de 1 à 10 atomes de carbone et de préférence 1 à 6 atomes de carbone, linéaire ou ramifié, contenant une fonction éther;
- R₄ est un atome d'hydrogène, un radical alkyle de 1 à 3 atomes de carbone ;
- R₅ est un atome d'hydrogène, un radical alkyle de 1 à 3 atomes de carbone ;
- ou bien R₄ et R₅ forment ensemble avec la liaison -N-C(=Y)- un cycle de type pyrrolidine, pyrrolidinone, piperidine ou piperidinone ;
- Y représente deux atomes d'hydrogène ou un hétéroatome comme l'oxygène, le soufre ;
- Ar représente un cycle 1,4-phényl, 2,5-pyridyl, 5,2-pyridyl ou 2,5-thiophényl ;
- X représente un atome d'oxygène éventuellement substitué par une chaîne alkyl ou alkylamine ou une liaison simple C-C;
- A représente un atome d'hydrogène ou la formule suivante : 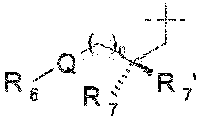 dans laquelle,
▪ Q est un atome d'oxygène ou la liaison -NH-;
▪ R₆ représente un atome d'hydrogène, un radical alkyle de 1 à 6 atomes de carbone, un radical cycloalkyle de 3 à 6 atomes de carbone, un radical -C(O)CH₃ ou -C(O)CH₂CH₃ ;
▪ R₇ et R₇' représentent, indépendamment l'un de l'autre, un atome d'hydrogène ou un groupement hydroxyle, à la condition que R₇ et R₇' ne soient pas simultanément un groupement hydroxyle ;
▪ n vaut 0, 1, 2, 3, 4 ou 5 ;
et les sels des composés de formule (I) lorsque R₃ représente un atome d'hydrogène ainsi que les isomères géométriques desdits composés de formule (I).

4. Composition selon l'une des revendications 1 à 3 **caractérisée en ce que** le rétinoïde est l'acide 3"-tert-butyl-4'-(2-hydroxy-ethoxy)-4"-pyrrolidin-1-yl-[1,1';3',1"]-terphenyl-4-carboxylique.

5. Composition selon l'une des revendications 1 à 4 **caractérisée en ce que** la silice hydrophobe est choisie dans la liste comprenant les silices Aérosil® R972, R974, R104, R106, R202, R805, R812, R812S, R816, R7200, R8200, R9200 et R711, les silices pyrogéniques hydrophobes HDK 13L et H2000 et leurs mélanges.

6. Composition selon l'une des revendications 1 à 5 **caractérisée en ce que** l'agent gélifiant est choisi parmi les polymères d'origine végétale, les gommes, les pectines, la cellulose et ses dérivés, les polymères d'origine microbiologiques tel que la gomme xanthane, et les polymères gélifiants d'origine synthétique, et de préférence dans la liste comprenant :
- les « Acrylates/C10-30 Alkyl Acrylate Crosspolymer » vendus sous le nom de Pemulen TR-1 ou Pemulen TR-2,
- les gélifiants de la famille des polyacrylamides comme le mélange Sodium acrylamide/acryloyldimethyltaurate copolymer / isohexadecane / polysorbate 80 vendu sous le nom de Simulgel 600PHA,
- le mélange polyacrylamide/isoparaffine C13-14/laureth-7 vendu sous le nom de Sepigel 305,
- les carbomers vendus sous le nom d'Ultrez 20®, d'Ultrez 10®, de Carbopol 1382® ou de Carbopol ETD2020NF®, de carbopol 981 ou encore carbopol 980,
- les polysaccharides avec à titre d'exemples non limitatifs la gomme de xanthane telle que le Xantural 180®, la gellan gum vendu sous le nom de Kelcogel, la gomme guar, la cellulose et ses dérivés tel que la microcrystalline cellulose et carboxymethyl cellulose de sodium vendue sous le nom d'Avicel CL-611, l'hydroxypropylmethylcellulose en particulier le produit vendu sous le nom de Methocel E4M premium ou l'hydroxyéthylcellulose, en particulier, le produit vendu sous le nom de Natrosol HHX 250®, la sodium carboxymethylcellulose en particulier la Blanose cellulose gum 7F,
- la famille des aluminium magnésium silicates tel que le Veegum K,
- la famille des polymères acryliques couplés à des chaînes hydrophobes tel que le PEG-150/decyl/SMDI copolymer vendu sous le nom de Aculyn 44,
- la famille des amidons modifiés tels que l'amidon de pomme de terre modifié vendu sous le nom de Structure Solanace ou bien leurs mélanges,
- la famille des carraghénanes en particulier réparties sous quatre grandes familles : κ, λ, β, ω tel que les Viscarin®et les Gelcarin®, et
- leurs mélanges.

7. Composition selon l'une des revendications 1 à 6 **caractérisée en ce que** qu'elle comprend en outre au moins un agent conservateur.

8. Composition selon la revendication 7, **caractérisée en ce que** l'agent conservateur est choisi dans la liste comprenant le méthyl parabène le propyl parabène, le chlorure de benzalkonium, le phenoxyethanol, l'alcool benzylique, le sorbate de potassium, l'acide benzoique, le 2-Bromo-2-Nitropropane-1,3-Diol, la chlorhexidine, le chlorocrésol et ses dérivés, l'alcool éthylique et la diazolidinylurée.

9. Composition selon l'une des revendications 1 à 8 pour son utilisation en tant que médicament.

10. Procédé de préparation d'une composition pharmaceutique selon l'une quelconque des revendications 1 à 9 comprenant les étapes suivantes :
a) ajout de 20% à 80% en poids de la totalité de l'agent gélifiant dans la quantité totale d'eau,
b) pré-mélange à l'état solide du principe actif de la classe des rétinoïdes avec au moins une silice hydrophobe,
c) ajout à température ambiante et sous homogénéisation du pré-mélange obtenu à l'étape b) au gel liquide obtenu à l'étape a), et
d) addition sous homogénéisation de la quantité restante d'agent gélifiant sur le milieu obtenu à l'étape c).

11. Procédé selon la revendication 10 **caractérisé en ce que** un non solvant lipophile du principe actif de la classe des rétinoïdes est ajouté à l'étape b).

12. Composition pharmaceutique selon les revendications 1 à 9 pour son utilisation dans le traitement de pathologies dermatologiques liées à un désordre de la kératinisation portant sur la différenciation ou sur la prolifération cellulaire.

13. Composition pharmaceutique selon la revendication 12 pour son utilisation dans le traitement de pathologies dermatologiques du groupe comprenant :
1) les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) les troubles de la kératinisation, notamment les ichtyoses, les états ichtyosiformes, l'ichtyose lamellaire, la maladie de Darrier, les kératodermies palmoplantaires, les leucoplasies, le pityriasis rubra pilaire et les états leucoplasiformes, le lichen cutané ou muqueux (buccal) ;
3) les affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore la dermatite atopique et les différentes formes d'eczema;
4) les désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose, les pigmentations et les rides ;
5) Toute condition liée à des proliférations dermiques ou épidermiques bénignes, qu'elles soient ou non d'origine virale telles que verrues vulgaires, les verrues planes , le molluscum contagiosum et l'épidermodysplasie verruciforme, les papillomatoses orales ou florides,;
6) les désordres dermatologiques tels que les dermatoses immunes comme le lupus érythémateux, les maladies immunes bulleuses et les maladies du collagène, telle la sclérodermie ;
7) les stigmates de l'atrophie épidermique et/ou dermique induite par les corticostéroïdes locaux ou systémiques, ou toute autre forme d'atrophie cutanée,
8) les troubles de la cicatrisation, ou pour prévenir ou pour réparer les vergetures, ou encore pour favoriser la cicatrisation,
9) dans le traitement de toute affection d'origine fongique au niveau cutané tel que le tinea pedis et le tinea versicolor,
10) les désordres de la pigmentation, tel l'hyperpigmentation, le mélasma, l'hypopigmentation ou le vitiligo ;
11) les états cancéreux ou précancéreux, cutanés ou muqueux comme les kératoses actiniques, la maladie de Bowen, les carcinomes in-situ, le kératoacanthome et les cancers cutanés comme le carcinome basocellulaire (BCC), le carcinome spinocellulaire (SCC) et les lymphomes cutanés tel que le lymphome T.

## Patentansprüche

1. Pharmazeutische Zusammensetzung, umfassend
(a) einen Wirkstoff aus der Klasse der Retinoide,
(b) mindestens ein Geliermittel,
(c) mindestens eine hydrophobe Kieselsäure und
(d) Wasser.

2. Zusammensetzung nach Anspruch 1, **dadurch gekennzeichnet, dass** der Wirkstoff aus der Klasse der Retinoide in dispergierter Form vorliegt.

3. Zusammensetzung nach Anspruch 1 oder Anspruch 2, **dadurch gekennzeichnet, dass** das Retinoid ausgewählt ist aus einer der Verbindungen, repräsentiert durch die folgende allgemeine Formel (I): 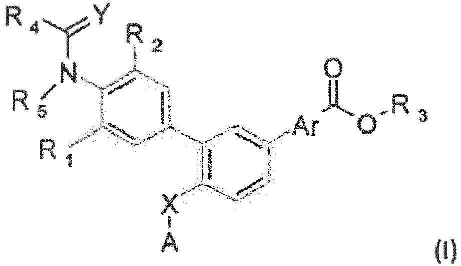 worin:
- R₁ ein Wasserstoffatom, ein Alkylrest mit 1 bis 4 Kohlenstoffatomen oder ein -CF₃-Rest ist;
- R₂ ein Wasserstoffatom, ein Alkyl- oder Alkoxyrest mit 1 bis 4 Kohlenstoffatomen oder ein Chloratom ist;
- R₃ ein Wasserstoffatom, ein Alkyl- oder Alkoxyrest mit 1 bis 10 Kohlenstoffatomen und vorzugsweise 1 bis 6 Kohlenstoffatomen, der linear oder verzweigt ist und gegebenenfalls durch eine Methoxygruppe substituiert ist, oder ein Alkylrest von 1 bis 10 Kohlenstoffatomen und vorzugsweise 1 bis 6 Kohlenstoffatomen, der linear oder verzweigt ist und eine Etherfunktion enthält, ist;
- R₄ ein Wasserstoffatom, ein Alkylrest mit 1 bis 3 Kohlenstoffatomen ist;
- R₅ ein Wasserstoffatom, ein Alkylrest mit 1 bis 3 Kohlenstoffatomen ist;
- oder R₄ und R₅ zusammen mit der Bindung -N-C(=Y)-einen Ring vom Pyrrolidin-, Pyrrolidinon-, Piperidin- oder Piperidinon-Typ bilden;
- Y für zwei Wasserstoffatome oder ein Heteroatom wie Sauerstoff, Schwefel, steht;
- Ar für einen 1,4-Phenyl-, 2,5-Pyridyl-, 5,2-Pyridyl- oder 2,5-Thiophenyl-Ring steht;
- X für ein Sauerstoffatom, das gegebenenfalls durch eine Alkyl- oder Alkylaminkette oder eine C-C-Einfachbindung substituiert ist, steht;
- A für ein Wasserstoffatom oder die folgende Formel steht: 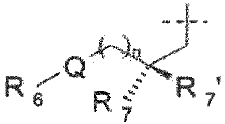 wobei
- Q ein Sauerstoffatom oder eine -NH-Bindung ist;
- R₆ für ein Wasserstoffatom, einen Alkylrest mit 1 bis 6 Kohlenstoffatomen, einen Cycloalkylrest mit 3 bis 6 Kohlenstoffatomen, einen Rest -C(O)CH₃ oder -C(O)CH₂CH₃ steht;
- R₇ und R₇' unabhängig voneinander für ein Wasserstoffatom oder eine Hydroxylgruppe stehen, mit der Maßgabe, dass R₇ und R₇' nicht gleichzeitig eine Hydroxylgruppe sind;
- n 0, 1, 2, 3, 4 oder 5 ist;
und den Salzen der Verbindungen der Formel (I), wenn R₃ für ein Wasserstoffatom steht, sowie den geometrischen Isomeren der Verbindungen der Formel (I).

4. Zusammensetzung nach einem der Ansprüche 1 bis 3, **dadurch gekennzeichnet, dass** das Retinoid 3"-*tert-*Butyl-4'-(2-hydroxyethoxy)-4"-pyrrolidin-1-yl-[1,1';3',1"]-terphenyl-4-carbonsäure ist.

5. Zusammensetzung nach einem der Ansprüche 1 bis 4, **dadurch gekennzeichnet, dass** die hydrophobe Kieselsäure aus der Liste ausgewählt ist, umfassend die Kieselsäuren Aerosil® R972, R974, R104, R106, R202, R805, R812, R812S, R816, R7200, R8200, R9200 und R711, die hydrophoben pyrogenen Kieselsäuren HDK 13L und H2000 und Mischungen davon.

6. Zusammensetzung nach einem der Ansprüche 1 bis 5, **dadurch gekennzeichnet, dass** das Geliermittel ausgewählt ist aus Polymeren pflanzlichen Ursprungs, Gummen, Pektinen, Cellulose und ihren Derivaten, Polymeren mikrobiologischen Ursprungs, wie Xanthangummi, und gelierenden Polymeren synthetischen Ursprungs, und vorzugsweise aus der Liste, die Folgendes umfasst:
- "Acrylate/C10-30-Alkylacrylat-Crosspolymer", verkauft unter dem Namen Pemulen TR-1 oder Pemulen TR-2,
- Gelbildner der Familie der Polyacrylamide, wie die Mischung Natriumacrylamid/Acryloyldimethyltaurat-Copolymer/Isohexadecan/Polysorbat 80, verkauft unter dem Namen Simulgel 600PHA,
- die Polyacrylamid/Isoparaffin C13-14/Laureth-7-Mischung, die unter dem Namen Sepigel 305 verkauft wird,
- die Carbomere, die unter dem Namen Ultrez 20®, Ultrez 10®, Carbopol 1382® oder Carbopol ETD 2020NF®, Carbopol 981 oder Carbopol 980 verkauft werden,
- Polysaccharide, wie, als nicht beschränkende Beispiele, Xanthangummi, wie Xantural 180®, Gellangummi, verkauft unter dem Namen Kelcogel, Guargummi, Cellulose und ihre Derivate, wie mikrokristalline Cellulose und Natriumcarboxymethylcellulose, verkauft unter dem Namen Avicel CL-611, Hydroxypropylmethylcellulose, insbesondere das unter dem Namen Methocel E4M Premium verkaufte Produkt, oder Hydroxyethylcellulose, insbesondere das unter dem Namen Natrosol HHX 250® verkaufte Produkt, Natriumcarboxymethylcellulose, insbesondere Blanose Cellulose Gummi 7F,
- die Familie der Aluminiummagnesiumsilikate, wie Veegum K,
- die Familie der Acrylpolymere, die an hydrophobe Ketten gekoppelt sind, wie das PEG-150/Decyl/SMDI-Copolymer, das unter dem Namen Aculyn 44 verkauft wird,
- die Familie der modifizierten Stärken, wie die modifizierte Kartoffelstärke, die unter dem Namen Structure Solanace verkauft wird, oder deren Mischungen,
- die Carrageen-Familie, insbesondere wie in vier Hauptfamilien unterteilt: κ, λ, β, ω, wie Viscarin® und Gelcarin®, und
- Mischungen davon.

7. Zusammensetzung nach einem der Ansprüche 1 bis 6, **dadurch gekennzeichnet, dass** sie weiterhin mindestens ein Konservierungsmittel enthält.

8. Zusammensetzung nach Anspruch 7, dadurch gekenneichnet, dass das Konservierungsmittel aus der Liste ausgewählt ist, die Methylparaben Propylparaben, Benzalkoniumchlorid, Phenoxyethanol, Benzylalkohol, Kaliumsorbat, Benzoesäure, 2-Brom-2-nitropropan-1,3-diol, Chlorhexidin, Chlorcresol und seine Derivate, Ethylalkohol und Diazolidinylharnstoff umfasst.

9. Zusammensetzung nach einem der Ansprüche 1 bis 8 zur Verwendung als Medikament.

10. Verfahren zur Herstellung einer pharmazeutischen Zusammensetzung nach einem beliebigen der Ansprüche 1 bis 9, umfassend die folgenden Schritte:
a) Zusetzen von 20 Gew.-% bis 80 Gew.-% der Gesamtmenge von Gelierungsmittel in der Gesamtmenge an Wasser,
b) Vormischen des Wirkstoffs aus der Klasse der Retinoide im Festzustand mit mindestens einer hydrophoben Kieselsäure,
c) Zusetzen der in Schritt b) erhaltenen Vormischung zu dem in Schritt a) erhaltenen flüssigen Gel bei Raumtemperatur und unter Homogenisieren, und
d) Zusetzen der Restmenge des Gelierungsmittels zu dem in Schritt c) erhaltenen Medium unter Homogenisieren.

11. Verfahren nach Anspruch 10, **dadurch gekennzeichnet, dass** ein lipophiles Nicht-Lösungsmittel des Wirkstoffs aus der Klasse der Retinoide bei Schritt b) zugesetzt wird.

12. Pharmazeutische Zusammensetzung nach den Ansprüchen 1 bis 9 zur Verwendung bei der Behandlung von dermatologischen Pathologien, die mit einer Keratinisierungs-Störung betreffend die Differenzierung oder Zellproliferation verbunden sind.

13. Pharmazeutische Zusammensetzung nach Anspruch 12 zur Verwendung bei der Behandlung von dermatologischen Erkrankungen der Gruppe, die Folgendes umfasst:
1) dermatologische Leiden, die mit einer Keratinisierungs-Störung betreffend die Differenzierung oder Zellproliferation verbunden sind, insbesondere zur Behandlung von Acne vulgaris, comedonica, polymorpha, rosacea, Acne nodulocystica, conglobata, Acne senilis, sekundärer Akne, wie Sonnen-, medikamentöser oder professioneller Akne;
2) Keratinisierungs-Probleme, insbesondere Ichthyosen, ichthyosiforme Zustände, lamellare Ichthyosis, Darier-Krankheit, Keratosis palmoplantaris, Leukoplakien, Pityriasis rubra pilaris und leucoplasiforme Zustände und kutaner oder muköser Lichen (bukkal);
3) dermatologische Leiden mit einer entzündlichen immunoallergischen Komponente, mit oder ohne Zellproliferationsstörung, und insbesondere alle Formen von Psoriasis, entweder der Haut, Schleimhaut oder Nägel, und weiterhin psoriatischer Rheumatismus, oder alternativ atopische Dermatitis und verschiedene Formen von Ekzem;
4) Hautstörungen wegen Exposition gegenüber UV-Strahlung, sowie für die Reparatur oder Bekämpfung von Hautalterung, ob lichtinduziert oder chronologisch, oder zum Reduzieren von Pigmentierungen und aktinischen Keratosen oder sonstigen Pathologien, die mit dem chronologischen oder aktinischen Altern verbunden sind, wie wie Xerose, Pigmentierungen und Falten;
5) jedwede Zustände, die verbunden sind mit dermalen oder epidermalen benignen Proliferationen, ob viralen Ursprungs oder nicht, wie gemeine Warzen, Flachwarzen, Molluscum contagiosum und Epidermodysplasie verruciformis, Papillomatosis oralis oder florida;
6) dermatologische Störungen, wie Immun-Dermatosen, wie Lupus erythematodes, bullöse Immunerkrankungen und Kollagenkrankheiten, wie Sklerodermie;
7) Stigmata der Epidermis- und/oder Dermis-Atrophie, induziert durch lokale oder systemische Kortikosteroide, oder jede andere Form von Hautatrophie,
8) Vernarbungs- bzw. Heilungsprobleme oder zum Verhindern oder Reparieren von Dehnungsstreifen, oder zum Fördern der Heilung,
9) Behandlung jedweden Zustands fungalen Ursprungs auf Ebene der Haut, wie Tinea pedis und Tinea versicolor,
10) Störungen der Pigmentierung, wie Hyperpigmentation, Melasma, Hypopigmentierung oder Vitiligo;
11) kanzeröse oder präkanzeröse Zustände, kutan oder mukös, wie aktinische Keratosen, Bowen-Krankheit, In-situ-Karzinome, Keratoakanthom und Hautkrebsarten, wie Basalzellenkarzinom (BCC), Plattenepithelkarzinom (SCC) und kutane Lymphome, wie T-Zell-Lymphom.

## Claims

1. Pharmaceutical composition comprising
a) an active principle of the retinoid class,
b) at least one gelling agent,
c) at least one hydrophobic silica, and
d) water.

2. Composition according to Claim 1, **characterized in that** the active principle of the retinoid class is in dispersed form.

3. Composition according to Claim 1 or Claim 2, **characterized in that** the retinoid is chosen from one of the compounds represented by the general formula (I) below: 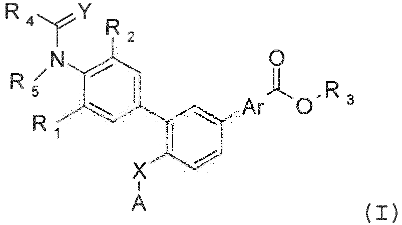 wherein
- R₁ is a hydrogen atom, an alkyl radical containing from 1 to 4 carbon atoms or a -CF₃ radical;
- R₂ is a hydrogen atom, an alkyl or alkoxy radical containing from 1 to 4 carbon atoms or a chlorine atom;
- R₃ is a hydrogen atom, a linear or branched alkyl or alkoxy radical containing from 1 to 10 carbon atoms and preferably 1 to 6 carbon atoms optionally substituted with a methoxy group, or a linear or branched alkyl radical containing from 1 to 10 carbon atoms and preferably 1 to 6 carbon atoms, containing an ether function;
- R₄ is a hydrogen atom or an alkyl radical containing from 1 to 3 carbon atoms;
- R₅ is a hydrogen atom or an alkyl radical containing from 1 to 3 carbon atoms;
- or else R₄ and R₅ form, together with the -N-C(=Y)-bond, a ring of pyrrolidine, pyrrolidinone, piperidine or piperidinone type;
- Y represents two hydrogen atoms or a heteroatom such as oxygen or sulfur;
- Ar represents a 1,4-phenyl, 2,5-pyridyl, 5,2-pyridyl or 2,5-thiophenyl ring;
- X represents an oxygen atom optionally substituted with an alkyl or alkylamine chain or a C-C single bond;
- A represents a hydrogen atom or the formula below: 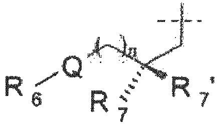 wherein
▪ Q is an oxygen atom or an -NH- bond;
▪ R₆ represents a hydrogen atom, an alkyl radical containing from 1 to 6 carbon atoms, a cycloalkyl radical containing from 3 to 6 carbon atoms, or a -C(O)CH₃ or -C(O)CH₂CH₃ radical;
▪ R₇ and R₇' represent, independently of one another, a hydrogen atom or a hydroxyl group, on condition that R₇ and R₇' are not simultaneously a hydroxyl group;
▪ n is 0, 1, 2, 3, 4 or 5;
and salts of the compounds of formula (I) when R₃ represents a hydrogen atom, and also the geometrical isomers of said compounds of formula (I).

4. Composition according to one of Claims 1 to 3, **characterized in that** the retinoid is 3"-*tert*-butyl-4'-(2-hydroxyethoxy)-4"-pyrrolidin-1-yl-[1,1';3',1"]-terphenyl-4-carboxylic acid.

5. Composition according to one of Claims 1 to 4, **characterized in that** the hydrophobic silica is chosen from the list comprising the silicas Aerosil® R972, R974, R104, R106, R202, R805, R812, R812S, R816, R7200, R8200, R9200 and R711, and the hydrophobic fumed silicas HDK 13L and H2000, and mixtures thereof.

6. Composition according to one of Claims 1 to 5, **characterized in that** the gelling agent is chosen from polymers of plant origin, gums, pectins, cellulose and derivatives thereof, polymers of microbiological origin such as xanthan gum, and gelling polymers of synthetic origin,
and preferably from the list comprising:
- the acrylates/C10-30 alkyl acrylate crosspolymer sold under the name Pemulen TR-1 or Pemulen TR-2,
- gelling agents of the polyacrylamide family, such as the sodium acrylamide/acryloyldimethyl taurate copolymer / isohexadecane / polysorbate 80 mixture sold under the name Simulgel 600PHA,
- the polyacrylamide/isoparaffin C13-14/laureth-7 mixture sold under the name Sepigel 305,
- the carbomers sold under the name Ultrez 20®, Ultrez 10®, Carbopol 1382® or Carbopol ETD2020NF®, Carbopol 981 or Carbopol 980,
- polysaccharides with, by way of nonlimiting examples, xanthan gum, such as Xantural 180®, the gellan gum sold under the name Kelcogel, guar gum, cellulose and its derivatives, such as the microcrystalline cellulose and sodium carboxymethylcellulose sold under the name Avicel CL-611, hydroxypropylmethylcellulose, in particular the product sold under the name Methocel E4M premium, or hydroxyethylcellulose, in particular the product sold under the name Natrosol HHX 250®, sodium carboxymethylcellulose, in particular Blanose cellulose gum 7F,
- the family of aluminum magnesium silicates such as Veegum K,
- the family of acrylic polymers coupled to hydrophobic chains, such as the PEG-150/decyl/SMDI copolymer sold under the name Aculyn 44,
- the family of modified starches, such as the modified potato starch sold under the name Structure Solanace, or mixtures thereof,
- the family of carrageenans, in particular divided up into four major families: κ, λ, β, ω, such as the Viscarin® products and the Gelcarin® products, and
- mixtures thereof.

7. Composition according to one of Claims 1 to 6, **characterized in that** it also comprises at least one preserving agent.

8. Composition according to Claim 7, **characterized in that** the preserving agent is chosen from the list comprising methyl paraben, propyl paraben, benzalkonium chloride, phenoxyethanol, benzyl alcohol, potassium sorbate, benzoic acid, 2-bromo-2-nitropropane-1,3-diol, chlorhexidine, chlorocresol and its derivatives, ethyl alcohol and diazolidinylurea.

9. Composition according to one of Claims 1 to 8, for its use as a medicament.

10. Process for preparing a pharmaceutical composition according to any one of Claims 1 to 9, comprising the following steps:
a) adding from 20% to 80% by weight of the total amount of the gelling agent to the total amount of water,
b) premixing in the solid state the active principle of the retinoid class with at least one hydrophobic silica,
c) adding, at room temperature and with homogenization, the premix obtained in step b) to the liquid gel obtained in step a), and
d) adding, with homogenization, the remaining amount of gelling agent to the medium obtained in step c) .

11. Process according to Claim 10, **characterized in that** a lipophilic non-solvent for the active principle of the retinoid class is added in step b).

12. Pharmaceutical composition according to Claims 1 to 9, for its use in treating dermatological pathologies associated with a keratinization disorder relating to cell differentiation or proliferation.

13. Pharmaceutical composition according to Claim 12, for use in the treatment of dermatological pathologies from the group comprising:
1) dermatological conditions associated with a keratinization disorder relating to cell differentiation and proliferation, in particular for treating common acne, comedonal acne, polymorphic acne, acne rosacea, nodulocystic acne, acne conglobata, senile acne, secondary acne such as solar acne, acne medicamentosa or occupational acne;
2) keratinization disorders, in particular ichthyosis, ichthyosiform conditions, lamellar ichthyosis, Darier's disease, palmoplantar keratoderma, leukoplakia, pityriasis rubra pilaris and leukoplakiform conditions, cutaneous or mucosal (buccal) lichen;
3) dermatological conditions with an inflammatory immunoallergic component, with or without a cell proliferation disorder, and in particular all forms of psoriasis, whether cutaneous, mucosal or ungual, and even psoriatic arthritis, or else atopic dermatitis and the various forms of eczema;
4) skin disorders caused by exposure to UV radiation, and also for repairing or combating skin aging, whether it is photo-induced or chronological, or for reducing actinic keratoses and pigmentations, or any pathological conditions associated with chronological or actinic aging, such as xerosis, pigmentations and wrinkles;
5) any condition associated with benign dermal or epidermal proliferations, whether or not they are of viral origin, such as common warts, flat warts, molluscum contagiosum and epidermodysplasia verruciformis, or oral or florid papillomatoses;
6) dermatological disorders such as immune dermatoses, for instance lupus erythematosus, bullous immune diseases and collagen diseases, such as scleroderma;
7) stigmata of epidermal and/or dermal atrophy induced by local or systemic corticosteroids, or any other form of cutaneous atrophy;
8) cicatrization disorders, or for preventing or repairing stretch marks, or else for promoting cicatrization;
9) in the treatment of any condition of fungal origin at the cutaneous level, such as tinea pedis and tinea versicolor;
10) pigmentation disorders, such as hyperpigmentation, melasma, hypopigmentation or vitiligo;
11) cutaneous or mucosal cancerous or precancerous conditions, such as actinic keratoses, Bowen's disease, in-situ carcinomas, keratoacanthomas and skin cancers such as basal cell carcinoma (BCC), squamous cell carcinoma (SCC) and cutaneous lymphomas such as T lymphoma.
